# EUROPEAN PATENT APPLICATION

(11) **EP 4 067 478 A1**
(43) Date of publication of application: **05.10.2022**
(21) Application number: 22162741.7
(22) Date of filing: 03.11.2015
(51) Int. Cl.: C12N 5/00, C14C 13/00, D06N 3/00

(54) **REINFORCED ENGINEERED BIOMATERIALS AND METHODS OF MANUFACTURE THEREOF**

(30) Priority: 03.11.2014 US 201462074507 P; 21.09.2015 US 201562221521 P
(62) Divisional of application: 20158046.1
(71) Applicant: Modern Meadow, Inc., Nutley, New Jersey 07110 (US)
(72) Inventor: FORGACS, Gabor, Nutley, 07110 (US); PURCELL, Brendan, Patrick, Nutley, 07110 (US); JAKAB, Karoly, Robert, Nutley, 07110 (US)
(74) Representative: Maiwald Patent- und Rechtsanwaltsgesellschaft mbH

(57) **Abstract**

Reinforced engineered biomaterials, and methods of making them, may be useful to form engineered leathers. Such engineered hides may be reinforced by encapsulating, and/or cross-linking to, a mesh and/or scaffold material, which may be a synthetic polymeric material. The resulting hide may include a stratified structure in which the mesh is held between layers of collagen-dense regions, where the collagen-dense regions overlaying the mesh following the contour of the mesh. Alternatively or additionally, the resulting hide may be a composite of a fibrous matrix that has been tanned to allow crosslinking of the fibrous matrix to the collagen formed by cultured cells. These engineered leathers may be referred to as fiber-reinforced biological tissue composites. The resulting materials may have superior durability and flexibility compared to other engineered materials and native leather, while retaining the look and feel of native leather.

## Description

### CROSS REFERENCE TO RELATED APPLICATIONS

This patent application claims priority to U.S. provisional patent application no. 62/074,507, filed on 11/3/2014, titled "REINFORCED ENGINEERED BIOMATERIALS AND METHODS OF MANUFACTURE THEREOF"; and U.S. provisional patent application no. 62/221,521, filed on 9/21/2015, and titled "FIBER REINFORCED TISSUE COMPOSITES".

This application may be related to U.S. Patent Application No. 13/853,001, filed on Mar. 28, 2013 (titled "ENGINEERED LEATHER AND METHODS OF MANUFACTURE THEREOF"), published as US-2013-0255003. This application may also be related to International Patent Application No. PCT/US2014/042384 ("ENGINEERED LEATHER AND METHODS OF MANUFACTURE THEREOF"), filed on Jun. 13, 2014, and published as WO2014/201406, which claims priority to U.S. Provisional Patent Application No. 61/834,867 filed on Jun. 13, 2013, and titled "ENGINEERED LEATHER AND METHODS OF MANUFACTURE THEREOF". Each of these patent applications is herein incorporated by reference in its entirety.

### INCORPORATION BY REFERENCE

All publications and patent applications mentioned in this specification are herein incorporated by reference in their entirety to the same extent as if each individual publication or patent application was specifically and individually indicated to be incorporated by reference.

### FIELD

Described herein are methods for making engineered leather that incorporates a reinforcing structure, as well as the resulting leather formed by these methods. These engineered leathers may be used in place of, or in combination with, animal hide leathers.

### BACKGROUND

Leather is used in a vast variety of applications, including furniture upholstery, clothing, shoes, luggage, handbag and accessories, and automotive applications. Currently, skins of animals are used as raw materials for natural leather. However, skins from livestock pose environmental concerns because raising livestock requires enormous amounts of feed, pastureland, water, and fossil fuel. Livestock also produces significant pollution for the air and waterways. In addition, use of animal skins to produce leather is objectionable to socially conscious individuals. The global leather industry slaughters more than a billion animals per year. Most of the leather comes from countries with no animal welfare laws or have laws that go largely or completely unenforced. Leather produced without killing animals would have tremendous fashion novelty and appeal.

Although synthetic leather was developed to address some of these concerns, it lacks the quality, durability, and prestige of natural leather. Thus far, scientifically sound and industrially feasible processes have not been developed to produce natural leather. Accordingly, there is a need for a solution to demands for alternative to leather produced from live animals. There is a need for a alternatives to leather produced from live animals, and in particular, there is a need for durable and sustainably-producible leather material.

Natural leather is typically a durable and flexible material created by the tanning of animal rawhide and skin, often cattle hide. Tanning is generally understood to be the process of treating the skins of animals to produce leather. Tanning may be performed in any number of well-understood ways, including vegetable tanning (e.g., using tannin), chrome tanning (chromium salts including chromium sulfate), aldehyde tanning (using glutaraldehyde or oxazolidine compounds), syntans (synthetic tannins, using aromatic polymers), and the like.

Natural leather is typically prepared in three main parts: preparatory stages, tanning, and crusting. Surface coating may also be included. The preparatory stages prepare the hide/skin for tanning, and unwanted raw skin components are removed. The preparatory stages may include: preservation, soaking (rehydrating), liming, de-hairing, de-fleshing (removing subcutaneous material), splitting, reliming, deliming (to remove de-hairing and liming chemicals), bating (protein proteolysis), degreasing, frizzing, bleaching, pickling (changing pH), de-pickling, etc.

Tanning is performed to convert proteins in the hide/skin into a stable material that will not putrefy, while allowing the material to remain flexible. Chromium is the most commonly used tanning material. The pH of the skin/hide may be adjusted (e.g., lowered, e.g. to pH 2.8-3.2) to enhance the tanning; following tanning the pH may be raised ("basification" to a slightly higher level, e.g., pH 3.8-4.2).

Crusting refers to the post-tanning treatment that may include coloring (dying), thinning, drying or hydrating, and the like. Examples of crusting techniques include: wetting (rehydrating), sammying (drying), splitting (into thinner layers), shaving, neutralization (adjusting pH to more neutral level), retanning, dyeing, fatliquoring, filling, stuffing, stripping, whitening, fixation of unbound chemicals, setting, conditioning, softening, buffing, etc.

In practice, the process of converting animal skin into leather may include sequential steps such as: unhairing/dehairing, liming, deliming and bateing, pickling, tanning, neutralizing/Dyeing and Fat liquoring, drying and finishing. The dehairing process may chemically remove the hair (e.g., using an alkali solution), while the liming step (e.g., using an alkali and sulfide solution) may further complete the hair removal process and swell ("open up") the collagen. During tanning, the skin structure may be stabilized in the "open" form by replacing some of the collagen with complex ions of chromium. Depending on the compounds used, the color and texture of the leather may change. Tanned leather may be much more flexible than an untreated hide, and also more durable.

Skin, or animal hide, is formed primarily of collagen, a fibrous protein. Collagen is a generic term for a family of at least 28 distinct collagen types; animal skin is typically type 1 collagen (so the term collagen is typically assumed to be type 1 collagen), although other types of collagen may be used in forming leather. Collagens are characterized by a repeating triplet of amino acids, -(Gly-X-Y)*ₙ*-, so that approximately one-third of the amino acid residues are in collagen are glycine. X is often proline and Y is often hydroxyproline. Thus, the structure of collagen may consist of twined triple units of peptide chains of differing lengths. Different animals may produce different amino acid compositions of the collagen, which may result in different properties (and differences in the resulting leather). Collagen fiber monomers may be produced from alpha-chains of about 1050 amino acids long, so that the triple helix takes the form of a rod of about 300 nm long, with a diameter of 1.5 nm. In the production of extracellular matrix by fibroblast skin cells, triple helix monomers may be synthesized and the monomers may self-assemble into a fibrous form. These triple helices may be held together by salt links, hydrogen bonding, hydrophobic bonding, and covalent bonding. Triple helices can be bound together in bundles called fibrils, and fibril bundles come together to create fibers. Fibers typically divide and join with each other throughout a layer of skin. Variations of the crosslinking or linking may provide strength to the material. Fibers may have a range of diameters. In addition to type I collagen, skin (hides) may include other types of collagen as well, including type III collagen (reticulin), type IV collagen, and type VII collagen.

Previous attempts to make engineered leathers have proven unsuccessful or impractical. For example, EP1589098 ("the '098 application") describes a method of growing fibroblasts seeded onto three-dimensional bioactive scaffolds. The scaffolds may be made from collagen waste material from a tanning process ("split"), microparticles of pure collagen, particle of collagen waste material, or synthetic scaffolds (e.g., made of polymers such as HYAFF). The addition of the scaffold material complicates and increases the expense of their proposed process, and also affects the properties of any leather produced this way.

The '098 application is one example of a scaffolding technique for culturing leather, however method such as this, which use a scaffold of waste or engineered collagen materials, have not been widely used because they are costly and difficult to work with, and have proven technically difficult to work with and commercialize.

To date, the fabrication of leather by ex vivo (e.g., cell culture) techniques has proven difficult. One potential advantage of cultured (e.g., engineered) leather, is the possibility of adding materials, including synthetic materials during the manufacture process, so that such materials may be integrated into the resulting leather, for example, to provide increased strength, durability and functionality. Although references such as U.S. patent publication no. 2012/0023777 to Greene have suggested culturing leather by adding elements to cultured leather such as reinforcing scaffolds or spherical members, including hollow members, that may modify the density, weight and stiffness of the resulting material. Unfortunately, references such as this do not adequately describe how such materials may be manufactured, particularly as quantities and sizes necessary for meaningful commercial fabrication.

Described herein are engineered leathers that may include one or more reinforcing materials, which may be processed in a simple and highly robust manner that may be scale-able so that commercially relevant quantities and qualities of leather may be manufactured. The methods and materials described herein may address many of the problems of natural and previously-described engineered leathers including those identified above.

Also described herein are engineered leathers that may address the problems by forming materials akin to fiber reinforced composites (FRCs), in which cells are cultured on fibrous scaffolds (formed of fibers such as silk). In general, FRCs refer to composite building materials that form class of high performance materials used in a number of industries including energy, building, automotive, and sport. FRCs consist of a continuous matrix phase (typically a polymer matrix), a dispersed fiber phase (typically a stronger glass, carbon or cellulosic fiber), and an interface between the matrix and fibers. Within the interface, fibers are crosslinked with the matrix phase to bond the material together and transmit forces from the matrix phase to the stronger fibers. As a result, materials with incredible strengths can be realized that are not possible with each material individually.

As mentioned, numerous tissue engineering scaffolds have been developed over the past 25 years to build biological tissues with defined structures and dimensions. These scaffolds provide surface area for cells to adhere and grow tissue in three dimensions. Fibrous materials consisting of entangled or woven fibers, 100nm-100um in diameter, have been widely explored as tissue engineering scaffolds due to their large surface areas for cell growth per unit volume, and high porosities to allow cell infiltration throughout the 3D scaffold architecture. Typically, tissue engineering scaffolds are biodegradable allowing the tissue to replace the scaffold as it grows. Therefore, the final product consists only of biological tissue to improve biocompatibility following implantation.

Tissue engineering constructs are generally grown for biomedical applications, including insertion into a body to repair and/or replace biological tissue, thus biocompatibility has been an important consideration. However, the use of biological tissues for consumer applications requires a much different set of considerations. In such cases, the durability, appearance and ability to be tanned or preserved must be considered. Described herein are methods and techniques for the fabrication of biological tissue, as well as the resulting engineered material, that may address the concerns described above. In particular, described herein are composites wherein the tissue is grown throughout a fibrous scaffold and crosslinked to the scaffold during a process analogous to tanning in order to create a novel class of high performance composites, as well as methods of forming such composites. These engineered leathers may replicate much of the structures and properties of natural leathers, but may be processed in a much simpler manner.

### SUMMARY OF THE DISCLOSURE

Described herein are reinforced engineered hides, and methods of making these hides. These hides may be tanned to form leather materials, which may be used in any place that natural leather is used, including to fabricate articles for wearing (clothing, shoes, etc.), furniture (e.g., upholstery) or the like (belts, wristbands, etc.).

For example, a method of making a reinforced engineered hide may include: placing a first plurality of collagen-releasing cells on a first surface of a mesh, the mesh having a first surface and a second surface; culturing the first plurality of collagen-releasing cells to form a first layer of collagen covering the first surface; flipping the mesh over and placing a second plurality of collagen-releasing cells onto the second surface of the mesh; and culturing the second plurality of collagen-releasing cells to form a second layer of collagen covering the second surface, wherein the mesh is embedded between the first and second layers of collagen and the first and second layers of collagen are connected to each other through the mesh.

In general, placing the cells may include placing (e.g., seeding) single cells, cell clumps/clusters, and/or layers/sheets of cells within a collagen sheet that the cells have secreted. The cells may be placed directly onto the surface, including onto the surface of the mesh or previously placed layer.

In any of the variations described herein the mesh may be (releasably) held in a frame. The frame may be manipulated to transfer, including flipping, the mesh and any cell/collagen layers thereon. For example, placing the first plurality of collagen-releasing cells may comprise placing the first plurality of collagen-releasing cells on the first surface of the mesh wherein the mesh is releasably held in a frame. The frame may remain on the cells during the entire process of making the hide, and may be removed before, or in some variations after, tanning.

Any of these methods may include flipping the mesh over one or more times during the formation of the material, to apply additional layers of cells (and therefore collagen) to the growing material. For example, the method may include flipping the mesh over so that the second surface is facing up and placing at least one first additional plurality of collagen-releasing cells atop the second surface and culturing the at least one first additional plurality of collagen-releasing cells to form at least one first additional layer of collagen covering the second surface. Alternatively or additionally, the method may include flipping the mesh over so that the first surface is facing up and placing at least one second additional plurality of collagen-releasing cells atop the first surface and culturing the at least one second additional plurality of collagen-releasing cells to form at least one second additional layer of collagen over the second surface.

Thus, for example, the method may include sequentially flipping the mesh over and placing at least one additional plurality of collagen-releasing cells atop the first surface or second surface and culturing the at least one second additional plurality of collagen-releasing cells to form at least one additional layer of collagen over the first or second surface.

As mentioned, the material may be tanned to form a leather. For example, the method may include tanning the material including the mesh embedded between the first and second layers of collagen. Tanning may include tanning the material having the mesh embedded between the first and second layers of collagen with mesh removably held by a frame, wherein the mesh was secured to the frame during the placement steps (e.g., the step of placing the first plurality of collagen-releasing cells on the first surface of the mesh).

As mentioned, in some variations, placing the cells may include placing a sheet of cells (and collagen). A sheet of collagen may be formed by culture the cells in a culture dish (or on another piece of mesh) and transferring the sheet onto the growing material including the mesh.

In some variations the method includes placing the mesh onto a preliminary layer of collagen-releasing cells in a culture dish so that the second surface of the mesh rests over the preliminary layer prior to placing the first plurality of collagen-releasing cells on the first surface of the mesh. This method may be particularly helpful in variations in which the mesh has relatively large pore sizes (e.g., the spacings through the mesh are greater than or equal to about 0.5 mm). Similarly, in some variations the method may include placing a plug against the second surface of the mesh before placing the first plurality of collagen-releasing cells on the first surface of the mesh.

Any of the methods described herein may include using a mesh formed of a material that the cells do not typically adhere to; despite not adhering, which may make it difficult for the cells to grow and release collagen, the cells may release collagen over the mesh. This may be possible in some variations, because of the relatively large pore sizes (e.g., greater than diameter of the cells) so that the cells adhere through the mesh openings (e.g., to a substrate, plug, or other collagen/cell layer).

A method of making a reinforced engineered hide may include: placing a first plurality of collagen-releasing cells on a first surface of a mesh, the mesh having a first surface and a second surface; culturing the first plurality of collagen-releasing cells to form a first layer of collagen covering the entire first surface; flipping the mesh over and placing a second plurality of collagen-releasing cells onto the second surface of the mesh; culturing the second plurality of collagen-releasing cells to form a second layer of collagen covering the entire second surface, wherein the mesh is embedded between the first and second layers of collagen and the first and second layers of collagen are connected to each other through the mesh; and sequentially forming additional layers of collagen over the first, second or first and second surfaces of the mesh to form a reinforced engineered hide having the mesh sandwiched between a plurality of layers of collagen-dense regions.

Sequentially forming additional layers of collagen may include forming a reinforced engineered hide having the striated pattern including a plurality of layers of collagen-dense regions and at least one mesh layer.

A method of making a reinforced engineered hide may include: placing a first plurality of collagen-releasing cells on a first surface of a mesh, the mesh having a first surface and a second surface, wherein the mesh is releasably held in a frame; culturing the first plurality of collagen-releasing cells to form a first layer of collagen covering the first surface; flipping the frame and the mesh over and placing a second plurality of collagen-releasing cells onto the second surface of the mesh; culturing the second plurality of collagen-releasing cells to form a second layer of collagen covering the second surface so that the mesh is embedded between the first and second layers of collagen and the first and second layers of collagen are connected to each other through the mesh; placing at least one first additional plurality of collagen-releasing cells atop the second surface and culturing the at least one first additional plurality of collagen-releasing cells to form at least one first additional layer of collagen covering the second surface; flipping the frame and mesh over and placing at least one second additional plurality of collagen-releasing cells atop the first surface and culturing the at least one second additional plurality of collagen-releasing cells to form at least one second additional layer of collagen over the second surface; and removing the mesh from the frame.

Also described herein are any of the reinforced engineered hides made by the methods described. For example, a reinforced engineered hide may include: a body extending in a plane and having a volume normal to the plane, the volume comprising a plurality of layers of collagen-dense regions extending in the plane within the volume to form a stratified pattern of collagen dense-regions; and a reinforcing mesh within the planar body and extending in the plane of the body, wherein a first layer of the plurality of layers of collagen-dense regions is adjacent to the mesh on a first side and follows a contour of the first side of the mesh and a second layer of the plurality of layers of collagen-dense regions is adjacent to the mesh on a second side and follows a contour of the second side of the mesh. The hides may be tanned.

The hides may be substantially free of non-differentiated keratinocytes or epithelial cells. In particular, the hides may be free of any vascular tissue (e.g., blood vessels or the like), or remnants of these, as would be found in endogenous leather.

In general, the thickness of each layer of collagen-dense region may be between about 10 µm to about 200 µm (e.g., between about 20 µm to about 150 µm). The pore size of the mesh may be, for example, between about 2 µm to 5 mm.

In any of the reinforced engineered hides described herein the hide may include one or more colorants or pigments. Further, the hide may be patterned, e.g., including having a relief pattern; in some variations the pattern is the pattern of the mesh. In general, the mesh may comprise a flexible net-like structure having a pore size of less than 5 mm. In some variations the mesh comprises a synthetic polymeric material.

As an alternative or addition to the mesh reinforcement described herein, any of these methods and engineered fabrics (leathers) may include a fibrous matrix. The mesh may itself be a fibrous matrix, or may be formed of a fibrous matrix. Alternatively or additionally the mesh may be formed entirely (or in some variations partially) of a cross-linking material that can be cross-linked to the extracellular matrix grown by the cultured cells during the tanning process, as described herein. For example, the mesh may be formed of a protein fiber comprising amine, carboxylic acid and sulfhydryl groups.

For example, the engineered, reinforced leather materials (engineered leathers) described herein may also or alternatively include a composite of a fibrous matrix that has been tanned to allow crosslinking of the fibrous matrix to the collagen formed by cultured cells (e.g., fibroblasts). These engineered leathers may be referred to as fiber-reinforced biological tissue composites. Also described herein are methods of making such fiber-reinforced biological tissue composites.

For example, a method of forming a fiber-reinforced biological tissue composite may include forming the material by culturing tissue-producing cells (e.g., fibroblasts) on a fibrous scaffold having amine (-NH2) and carboxylic acid (-COOH) functional groups, which may be cross-linked (e.g., by tanning) to the tissue and/or proteins, such as collagen, formed and/or secreted by the cells and then tanning (e.g., chemically cross-linking). Tanning may be performed after the scaffold fibers have been at least partially covered in cultured cells and extracellular matrix released by the cultured cells.

In general, the scaffolds described herein are fibrous scaffolds formed of any cross-linkable material, but particularly protein materials (e.g., containing amine and carboxylic acid groups), such as silk. Silk is only one example of a fibrous scaffold that may be cross-linkable; silk is generally formed of a protein fiber that may be composed mainly of fibroin. For example, silk fibers from domesticated silkworms typically have a triangular cross section with rounded corners, 5-10 µm wide. The fibroin-heavy chain is composed mostly of beta-sheets, due to a 59-mer amino acid repeat sequence with some variations. Silkworm fibers are naturally extruded from two silkworm glands as a pair of primary filaments (brin), which are stuck together, with sericin proteins that act like glue, to form a bave. Bave diameters for tussah silk can reach 65 µm. Silk emitted by a silkworm may consist of two main proteins, sericin and fibroin, fibroin being the structural center of the silk, and serecin being the sticky material surrounding it. Fibroin is made up of the amino acids Gly-Ser-Gly-Ala-Gly-Ala and forms beta pleated sheets. Hydrogen bonds form between chains, and side chains form above and below the plane of the hydrogen bond network.

A mesh is generally a network of material (threads, stings, strands, fibers, etc.) that are connected, e.g., by weaving or otherwise. The mesh may have pores that are regular or irregular in size, and/or regular or irregular in shape, and/or regular or irregular in spacing, and/or regular or irregular in pattern. The mesh may be generally two-dimensional (e.g., forming a sheet or surface); three-dimensional meshes are also contemplated. As an example, pores may be between 10 nm and 5 cm in one or more of; diameter or spacing. For example, the pore size may be generally between a lower diameter of about 10 nm, 20 nm, 50 nm, 100 nm, 200 nm, 300 nm, 400 nm, 500 nm, .6 mm, .7 mm, .8 mm, .9 mm, 1 mm, 1.1 mm, 1.2 mm, 1.3 mm, 1.4 mm, 1.5 mm, 1.6 mm, 1.7 mm, 1.8 mm, 1.9 mm, 2 mm, 3 mm, 4 mm, 5 mm, 6 mm, 7 mm, 8 mm, 9 mm, 10 mm, 12 mm, 15 mm, 2 cm, 2.5 cm, 3 cm, 4 cm, 5 cm, etc., and an upper diameter of about 20 nm, 50 nm, 100 nm, 200 nm, 300 nm, 400 nm, 500 nm, .6 mm, .7 mm, .8 mm, .9 mm, 1 mm, 1.1 mm, 1.2 mm, 1.3 mm, 1.4 mm, 1.5 mm, 1.6 mm, 1.7 mm, 1.8 mm, 1.9 mm, 2 mm, 3 mm, 4 mm, 5 mm, 6 mm, 7 mm, 8 mm, 9 mm, 10 mm, 12 mm, 15 mm, 2 cm, 2.5 cm, 3 cm, 4 cm, 5 cm, 6 cm, 7 cm, 8 cm, 9 cm, 10 cm, etc., where the lower diameter is always less than the upper diameter. The stransds (e.g., fiber, threads, webs, etc.) or material forming the mesh may have diameters of between about 100nm and 5 mm in diameter (or in some cases more). A mesh may act as a scaffold; as used herein the scaffold may, but does not have to be, a mesh.

As mentioned, in any of the methods and apparatuses (e.g., engineered leathers) described herein, a scaffold may be a protein fiber containing amine and carboxylic acid groups, such as naturally occurring cellulose fibers containing (or modified to contain) amine and carboxylic acid groups. The fibers forming the scaffold may be chemically modified to enhance tissue crosslinking to scaffold. For example, the scaffold may be chemically modified to contain tissue crosslinking groups including amines, carboxylic acids, sulfates, aldehydes, hydrazides, sulfhydryls, epoxides, acrylates, etc. These tissue crosslinking groups may be protected during tissue growth and activated for crosslinking when tissue growth is complete. In particular, the crosslinking referred to herein is tanning, which may be identical to, or derived from, traditional tanning methods and techniques, including omitting those steps which are made unnecessary by the use of tissue culture as described herein. Further, an additional reinforcement crosslinking step can be used to crosslink chemistries not involved in the traditional tanning process (anything other than amine and carboxylic acid groups). Traditional tanning chemicals can then be used to give the fiber reinforced biological tissue composite a leather-like aesthetic. Scaffolds may also be formed or (and/or may include) carbon fibers, which may also be modified as discussed above.

In general, these tissue crosslinking groups may be pendant to the scaffold with a spacer between 10 daltons and 100 megadaltons. The scaffold may be crosslinked to the tissue through non-covalent interactions including ionic, hydrophobic and van der Waals forces. Alternatively or additionally, the scaffold may be cross-linked to the tissue through covalent bonds. For example, the scaffold may be directly reacted with amine or carboxylic acid groups on the tissue. The scaffold may be reacted with a crosslinker which reacts with amine or carboxylic acid groups on the tissue. The molecular weight of the crosslinker may be between 10 daltons and 100 megadaltons. The tissue referred to herein are the cultured cells and/or the products released by these cultured cells (e.g., extracellular matrix proteins, in particular collagen). Any of the crosslinkers described herein may include a functionality of the crosslinker of between 2 and 2000.

The scaffold may be composed of fibers. As mentioned, the fibers may be an appropriate size or dimension (e.g., the fibers may have a length of between about 100 nm to 1 m). The fibers may be assembled in a woven or non-woven architecture (or a combination of both). The density of fibers in the scaffold may be between 10 and 10,000 mg/cc. The porosity of the fibrous scaffold may be between 10 and 99%.

Any appropriate cells may be cultured on the fibrous scaffold. The cells may originate from a tissue and/or cell-line. For example, the cells may be of mammalian origin (e.g., bovine, porcine, ovine, etc.). The cells may be of reptile origin (e.g., snake, lizard, etc.). The cells may be of bird origin (e.g., chicken, ostrich, turkey, etc.). The cells may be of fish origin (e.g. shark, etc.). The cells may be of amphibian origin (e.g. frog, salamander, etc.). The cells may be genetically modified (e.g., to increase production of ECM, including, e.g., collagen, etc.) or they may be unmodified.

### BRIEF DESCRIPTION OF THE DRAWINGS

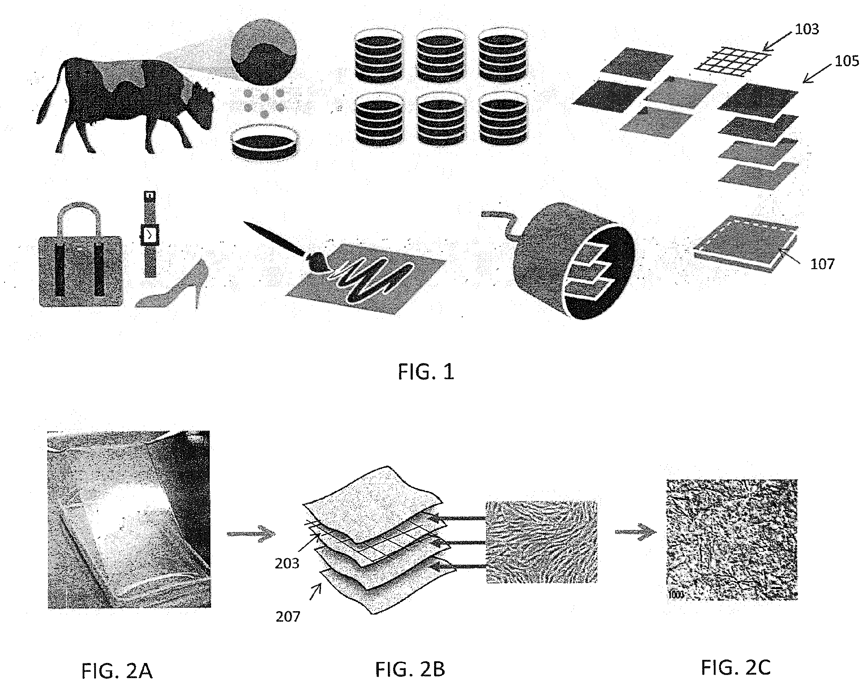

FIG. 1 shows an overview of one method of making engineered leather. In this example, cells are taken from an animal through a simple biopsy, and cells are then isolated and multiplied in a cell culture medium. The cells are then allowed (and/or induced) to produce collagen, as they would naturally after the cells are spread out so that the collagen forms sheets. The sheets are then treated to prevent contraction, and the thin non-contracting (e.g., decellularized) sheets are stacked on top of one another after seeding with additional collagen-forming cells, to form thicker sheets which are allowed to adhere by the action of the collagen-releasing cells. Finally, leather is formed from this multilayered structure through a shortened tanning process to modify the collagen.
FIGS. 2A-2C illustrate one example of forming a stack of sheets of collagen to create a reinforced engineered leather. In this example, a mesh material (any reinforcing material) may be added to within he stack/between the layers and the stacks (and/or individual cells seeded onto the layers) may be grown on top of the reinforcing material, thereby encapsulating the reinforcing material within the material. This technique may be particularly effective with large mesh-size openings.
FIG. 3A shows one example of a mesh material on a frame onto which cells may be grown
FIG. 3B schematically describes and exemplary method of making reinforced engineered leather, as described herein, in which cells are sequentially grown onto a first side of a mesh and the mesh (with grown cells and collagen) may be inverted to grow cells on the opposite side.
FIG. 4 is a schematic illustration of one variation of a method of growing reinforced leather.
FIG. 5 is another schematic illustration of a method of growing reinforced leather on a mesh.
FIG. 6 is another schematic illustration of a method of growing reinforced leather on a mesh.
FIG. 7 is a schematic illustration of a method of growing reinforced leather on a mesh.
FIGS. 8A and 8B show sectional and top views, respectively, of one example of a low-resolution section through an example of a reinforced leather manufactured using a methods such as the method diagrammatically illustrated in FIG. 4 or FIG. 5, above.
FIG. 8C shows the engineered leather of FIGS. 8A and 8B in which an outer layer of the material has been partially peeled off to reveal the mesh beneath.
FIG. 9 schematically illustrates a section through one example of the reinforced leather as described herein. The figure is not shown to scale.
FIG. 10 show a first view of an example of a fibrous scaffold formed of silk fibers that may be used to form the composite manufactured leather described herein. In FIG. 10 the image is shown at a low magnification (scale bar is 1 mm).
FIG. 11 shows the fibrous scaffold of FIG. 10 at higher magnification (scale bar is 0.5 mm).
FIGS. 12A and 12B illustrate tissue growth on a fibrous scaffold such as the silk scaffold shown in FIGS. 10A-10B. FIG. 12A shows the fibrous scaffold and FIG. 12B shows the fibrous scaffold of FIG. 12A following four weeks of fibroblast culture. Cells were seeded onto the silk fiber scaffold shown in FIG. 12A and after four weeks of culture the scaffold fibers are surrounded by tissue (FIG. 12B).
FIG. 13 shows an example of a silk scaffold onto which fibroblasts have been cultured, prior to tanning. In FIG. 13, a section of scaffold onto which fibroblasts have been cultured (e.g., for four weeks) has been stained with picrosirus red to visualize collagen.
FIG. 14 is a scanning electron micrograph showing a portion of a fibrous silk scaffold onto which fibroblasts have been grown and allowed (and in some variations stimulated) to secrete collagen. Collagen rich tissue has grown throughout the silk fiber scaffold.
FIG. 15 shows examples of four different composites of fibrous scaffolds (silk, high density PLLA, polyester, low density PLLA) and fibroblasts after eight weeks of culture, followed by tanning. Only the fibrous silk scaffold composite was successfully tanned, and only the fibrous silk scaffold includes amine and carboxylic acid groups which can crosslink with the tissue during the tanning process.
FIG. 16 shows an example of the leather-like surface of a fibrous silk scaffold composite after tanning.
FIG. 17 is an example of the edge of a fibrous silk scaffold composite after tanning, in which a gradient of tissue was produced towards the edge of the silk scaffold, revealing the silk fibers dispersed throughout the tissue matrix.

### DETAILED DESCRIPTION

In general, described herein are reinforced engineered biomaterials, and methods of making them. For example, described herein are engineered hides (e.g., leather) that incorporate a supporting material such as a mesh or scaffold embedded within the hide, as well as methods of making these reinforced engineered biomaterials.

In some variations the engineered hides described herein may be formed by forming sequential layers of collagen-releasing cells (e.g., allowed to grow and release collagen to form a layer) and combining one or more layers of collagen-releasing cells so that they may fuse with a support material such as a mesh. Alternatively or additionally, the engineered hides may be formed by adding collagen-releasing cells into a fibrous scaffold formed of a material that may be cross-lined during tanning directly to the released extracellular matrix (e.g., collagen). Thus, the mesh and/or scaffold may be treated or otherwise formed of a material that may be cross-linked to the ECM during tanning.

In some variations the engineered hides described herein may be formed by sequentially seeding collagen-releasing cells or layers of collagen-releasing cells onto a support material such as a mesh, and allowing the cells (or layer of cells) to grow and release collagen onto the support material. The cells or layers of cells are applied in a manner that allows them to receive nutrients; they may be allowed to mature to form the new layer (including a new layer of collagen) before forming a new layer on top of these cells.

In any of the methods described herein, the mesh may be provided as a support, and in forming the layer, the mesh material may be periodically flipped so that cells or pre-formed layers of cell may be placed on top of either the first or second side, and allowed to grow and adhere (releasing collagen) before flipping the material and the growing hide over to apply to the opposite side of the material.

As mentioned in the background section above, tissue engineering technology offers new opportunities to produce animal skin, hide (and leather therefrom) that are not associated with the environmental degradation of raising livestock. Tissue engineering has been defined as an interdisciplinary field that applies the principles of engineering and life sciences toward the development of biological substitutes that restore, maintain, or improve tissue function or a whole organ. Langer R, Vacanti JP, Tissue Engineering, Science 260(5110):920-926 (May 1993).

Tissue engineered products made using traditional materials and methods are limited in size due to the short distances gases and nutrients can diffuse to nourish interior cells, typically thicknesses of engineered constructs that are less than about 250-300 µm. Also, existing techniques fail to provide adequate speed and throughput for mass production of engineered products. As a result, existing tissue engineering methods result in unappealing thin sheets and pastes on a commercially infeasible scale. Thus, an objective of the animal skin/hide, or leather, and methods of making the same disclosed herein is to provide commercially viable and appealing animal skin, hide, or leather. Another objective is to provide high-throughput methods that reliably, accurately, and reproducibly scale up to commercial levels. Advantages of the animal skin, hide, or leather, and methods of making the same disclosed herein include, but are not limited to, production of customized tissues in a reproducible, high throughput and easily scalable fashion while keeping precise control of pattern formation, particularly in cases of multiple cell types, which may result in engineered animal skin, hide, or leather with appealing appearance, texture, thickness, and durability.

Disclosed herein are engineered animal hide and leather, and methods of producing the same. In certain embodiments, disclosed herein are engineered animal skins, hides, or leathers comprising one or a plurality of layers of animal cells comprising one or more types of skin cells, wherein said animal cells are cultured in vitro. In certain embodiments, each layer of animal cells provided herein is biofabricated. A structural scaffold, such as a mesh, may be incorporated into any of the engineered materials described herein.

Also described herein are engineered animal hide and leather and methods of producing them including a fibrous scaffold that may be used to grow cells that may release ECM which may be cross-linked to the fibrous scaffold during tanning. For example, the fibrous scaffold may include available reactive groups that may be cross-linked to one or more ECM proteins (e.g., collagen).

For example, the methods described herein may include first forming sheets of collagen by growing (culturing) collagen-releasing cells and allowing (and/or stimulating) them to secrete collagen. In culture the cells may be grown to confluence on a substrate such as the bottom of a cell culture dish, flask, roller, chamber (e.g., rotating chamber) or the like, and/or in a fibrous scaffold.

In general, the collagen-releasing cells described herein may be derived from tissue extracts/explants, immortalized cell lines, or manipulated (transgenic) cell lines, or any variation thereof. In some variations, the cell may be grown to complete confluence (e.g., 100% confluence), in which cells are inhibited from further growth but may continue to produce or be stimulated to produce and release collagen. In some variations, the cells may not be grown to complete confluence, (e.g., approximately 99% confluence, 95 % confluence, 90% confluence, 85% confluence, 80% confluence, etc.). Cells may be cultured until greater than 80% confluence, greater than 85% confluence, greater than 90% confluence, greater than 95% confluence and/or just under full (100%) confluence.

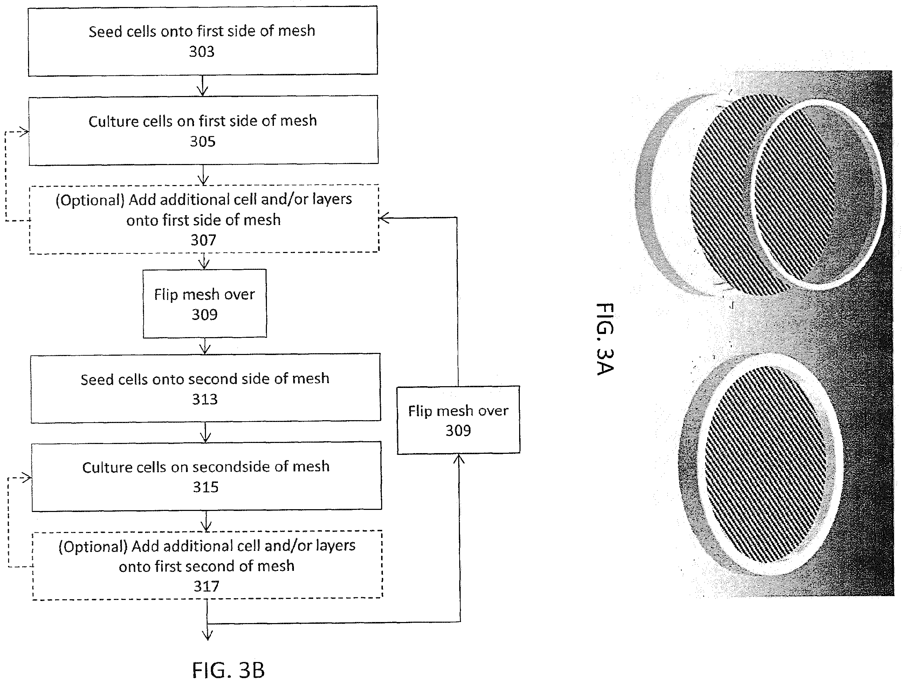

As described in greater detail below, and in particular reference to FIGS. 3-8C, cells and sheets of cells may be grown directly onto a support material (e.g., mesh); alternatively the cells may initially be grown in one or more sheets that are combined with a support material (e.g., mesh) and then allowed to grow further (e.g., by the additional of additional cells or sheet so cells that release collagen).

In general, the growing structures (e.g., sheets) including collagen may be treated to prevent contractions. Cultured cells, including collagen-releasing cells, may begin contracting during culture. The cultured cells forming the multiple sheets of collagen may be treated while growing, and/or towards the end of the growth. Typically, the sheets of collagen are treated before contraction begins, or before contraction above a threshold occurs. In general, it is desirable to prevent contractions because the contracting cells in a sheet of collagen may cause the sheet to deform, which may be undesirable when forming an engineered leather.

In some variations, treatment of the sheets of collagen or treatment of the cells forming the sheets of collagen) may include killing the cells. Thus the sheets of collagen including the cultured cells may be treated by decellularlizing (e.g., removing or killing) the collagen-releasing cells. In some variations, this may include treating the collagen sheets with ethanol. After treatment, the resulting sheets of collagen may be referred to as "non-contractile" sheets or "decellularized" sheets (for sheets treated to kill and/or remove the collagen-releasing cells).

After treating the sheets to form non-contractile sheets, the sheets may be washed, (e.g., rinsed, or the like) to remove the material used to treat the cells. Other additional treatments may also be performed. Thereafter, an engineered leather of a desired thickness may be formed using the non-contractile sheets. For example, the sheets may be stacked either sequentially or simultaneously, and adhered together. Adhesion may be achieved by again seeding the sheets (e.g., one surface of the sheet) so that collagen-releasing cells can communicate with one or both sides of the non-contractile sheets. This second group of cells may be the same cells used to grow the collagen sheets, or they may be different types of cells (though also collagen-releasing), or different distributions of cell types where multiple types of cells are used to grow the collagen sheets. A support material such as a mesh may be applied between sheets or on a single sheet onto which additional collagen-releasing cells are grown either by themselves or beneath another sheet.

At the addition of each new layer of cells or mesh and collagen-releasing cells (or layer of cells and released collagen), the second group of collagen-releasing cells may then be allowed to grow (e.g., be cultured) until sufficient adhesion is achieved between the two sheets. Sufficient adhesion may be determined by time in culture (e.g., about: 4 hours, 8 hours, 12 hours, 24 hours, 2 days, 3 days, 4 days, 5 days, 6 days, 7 days, etc.), or it may be empirically determined. For example the "stack" of sheets (two sheets being adhered together by the seeded second group of collagen-releasing cells) may be allowed to grow for more than about 4 days, more than about 5 days, more than about 6 days, more than about 7 days, more than about 8 days; and/or less than about 9 days, less than about 8 days, less than about 7 days, less than about 6 days, less than about 5 days, etc., expressly including between about 2-9 days, between about 4-7 days, about 5 days, etc.

In some variations a filler material may be inserted between the sheets before culturing them to allow them to adhere. Any appropriate filler material, but particularly collagenous filler material may be used. The filler material may be useful to increase the thickness of the stack forming the engineered leather, and/or reducing the number of collagen layers used to form an engineered leather. Example of filler materials include reconstituted collagen, and/or collagen pulp (e.g., mechanically sheered collagen). The reconstituted collagen may be depolymerized (e.g., collagen monomers or small polymers). Filler material may be added to a desired thickness. In some variations the filler material may be seeded with cells from the second group of collagen-releasing cells, in addition or alternatively to the collagen-releasing cells seeded onto one or both layers to be stacked onto each other around the filler material.

When adding additional sheets of collagen to a stack (e.g., non-contractile sheets of collagen) forming an artificial leather, additional sheets may be added either sequentially or in parallel, or a combination of both, in which a small number (e.g. less than about 8, less than about 7, less than about 6, less than about 5, less than about 4, less than about 3, 2, etc.) of sheets may be stacked atop a first of non-contractile sheet of collagen and the sheets may be allowed to adhere by culturing the second group of collagen-releasing cells placed between the two or more sheets (with or without filler material) with or without the reinforcing material (e.g., mesh). In this manner, a "stack" of collagen sheets that have been adhered may be formed. There after the dimensions (e.g., thickness) of the stack can be increased by combining two or more stacks and allowing them to adhere by culturing one (or more) atop the other with or without filler material after seeding them with some of the second group of collagen-releasing cells.

### Cells

Many cell types may be used for the collagen-releasing cells used to produce the sheets/layers of collagen, and thus the engineered skin, hide, and leather products described herein. The collagen-releasing cells may be homogenous (e.g., of the type of cell) or they may be a mixture of collagen-releasing cells and/or cells that release other ECM materials, and/or cells that do not release collagen. In some embodiments, the engineered animal skin, hide, and leather products are designed to resemble traditional animal skin, hide, and leather products and the cell types are chosen to approximate those found in traditional animal skin, hide, and leather products. In further embodiments, the engineered animal skin, hide, and leather products, and sheet/layers include animal epidermis, basement membrane, dermis, hypodermis, scale, scute, osteoderm, or a combination thereof. In some embodiments, animal epidermis provided herein comprises stratum corneum, stratum lucidum, stratum granulosum, stratum spinosum, stratum germinativum, stratum basale, or a combination thereof. In some embodiments, animal dermis provided herein comprises stratum papillare, stratum reticulare, or a combination thereof. In some embodiments, animal scale provided herein comprises placoid scale, cosmoid scale, ganoid scale, elasmoid scale, cycloid scale, ctenoid scale, crenate scale, spinoid scale, or a combination thereof.

In certain embodiments, animal cells provided herein comprise epithelial cells, fibroblasts, keratinocytes, comeocytes, melanocytes, Langerhans cells, basal cells, or a combination thereof. In some embodiments, epithelial cells provided herein comprise squamous cells, cuboidal cells, columnar cells, basal cells, or a combination thereof. In some embodiments, fibroblasts provided herein are dermal fibroblasts. In some embodiments, keratinocytes provided herein are epithelial keratinocytes, basal keratinocytes, proliferating basal keratinocytes, differentiated suprabasal keratinocytes, or a combination thereof.

In certain embodiments, the ratio of animal fibroblasts to animal keratinocytes provided herein is between about 20:1 to about 3:1. In some embodiments, the ratio of animal fibroblasts to animal keratmocytes provided herein is between about 20:1 to about 4:1. In some embodiments, the ratio of animal fibroblasts to animal keratmocytes provided herein is between about 20:1 to about 5:1. In some embodiments, the ratio of animal fibroblasts to animal keratmocytes provided herein is between about 20:1 to about 10:1. In some embodiments, the ratio of animal fibroblasts to animal keratmocytes provided herein is between about 20:1 to about 15:1. In some embodiments, the ratio of animal fibroblasts to animal keratmocytes provided herein is about 25:1. In some embodiments, the ratio of animal fibroblasts to animal keratmocytes provided herein is about 24:1. In some embodiments, the ratio of animal fibroblasts to animal keratmocytes provided herein is about 23:1. In some embodiments, the ratio of animal fibroblasts to animal keratmocytes provided herein is about 22:1. In some embodiments, the ratio of animal fibroblasts to animal keratmocytes provided herein is about 21:1. In some embodiments, the ratio of animal fibroblasts to animal keratmocytes provided herein is about 20:1. In some embodiments, the ratio of animal fibroblasts to animal keratmocytes provided herein is about 19:1. In some embodiments, the ratio of animal fibroblasts to animal keratmocytes provided herein is about 18:1. In some embodiments, the ratio of animal fibroblasts to animal keratmocytes provided herein is about 17:1. In some embodiments, the ratio of animal fibroblasts to animal keratmocytes provided herein is about 16:1. In some embodiments, the ratio of animal fibroblasts to animal keratmocytes provided herein is about 15:1. In some embodiments, the ratio of animal fibroblasts to animal keratmocytes provided herein is about 14:1. In some embodiments, the ratio of animal fibroblasts to animal keratmocytes provided herein is about 13:1. In some embodiments, the ratio of animal fibroblasts to animal keratmocytes provided herein is about 12:1. In some embodiments, the ratio of animal fibroblasts to animal keratmocytes provided herein is about 11:1. In some embodiments, the ratio of animal fibroblasts to animal keratmocytes provided herein is about 10:1. In some embodiments, the ratio of animal fibroblasts to animal keratmocytes provided herein is about 9:1. In some embodiments, the ratio of animal fibroblasts to animal keratmocytes provided herein is about 8:1. In some embodiments, the ratio of animal fibroblasts to animal keratmocytes provided herein is about 7:1. In some embodiments, the ratio of animal fibroblasts to animal keratmocytes provided herein is about 6:1. In some embodiments, the ratio of animal fibroblasts to animal keratmocytes provided herein is about 5:1. In some embodiments, the ratio of animal fibroblasts to animal keratmocytes provided herein is about 4:1. In some embodiments, the ratio of animal fibroblasts to animal keratinocytes provided herein is about 3:1. In some embodiments, the ratio of animal fibroblasts to animal keratinocytes provided herein is about 2:1.

In certain embodiments, animal cells provided herein are substantially free of non-differentiated keratinocytes, fibroblasts, or epithelial cells.

In other embodiments, the engineered animal skin, hide, or leather products include neural cells, connective tissue (including bone, cartilage, cells differentiating into bone forming cells and chondrocytes, and lymph tissues), epithelial cells (including endothelial cells that form linings in cavities and vessels or channels, exocrine secretory epithelial cells, epithelial absorptive cells, keratinizing epithelial cells, and extracellular matrix secretion cells), and undifferentiated cells (such as embryonic cells, stem cells, and other precursor cells), among others.

In certain embodiments, engineered animal skin, hide, or leather further comprises an extra¬cellular matrix or connective tissue. In certain embodiments, engineered animal skin, hide, or leather further comprises one or more components selected from the group consisting of collagen, keratin, elastin, gelatin, proteoglycan, dermatan sulfate proteoglycan, glycosoaminoglycan, fibronectin, laminin, dermatopontin, lipid, fatty acid, carbohydrate, and a combination thereof.

In some embodiments, the cells are obtained from commercial sources. In certain embodiments, the cells are derived from, by way of non-limiting examples, mammals, birds, reptiles, fish, crustaceans, mollusks, cephalopods, insects, non-arthropod invertebrates, and combinations thereof. In some embodiments, the animal cells human cells. In certain embodiments, the animal cells provided herein are non-human cells. In some embodiments, suitable cells are derived from mammals such as antelope, bear, beaver, bison, boar, camel, caribou, cat, cattle, deer, dog, elephant, elk, fox, giraffe, goat, hare, horse, ibex, kangaroo, lion, llama, lynx, mink, moose, oxen, peccary, pig, rabbit, seal, sheep, squirrel, tiger, whale, wolf, yak, and zebra, or combinations thereof. In some embodiments, suitable cells are derived from birds such as chicken, duck, emu, goose, grouse, ostrich, pheasant, pigeon, quail, and turkey, or combinations thereof.

In some embodiments, suitable cells are derived from reptiles such as turtle, snake, crocodile, and alligator, or combinations thereof.

In some embodiments, suitable cells are derived from fish such as anchovy, bass, catfish, carp, cod, eel, flounder, fugu, grouper, haddock, halibut, herring, mackerel, mahi mahi, manta ray, marlin, orange roughy, perch, pike, salmon, sardine, shark, snapper, sole, stingray, swordfish, tilapia, trout, tuna, and walleye, or combinations thereof.

In some embodiments, suitable cells are derived from amphibians such as frog, toad, salamander, newt, or combinations thereof.

In some embodiments, suitable cells are derived from crustaceans such as crab, crayfish, lobster, prawn, and shrimp, or combinations thereof.

In some embodiments, suitable cells are derived from mollusks such as abalone, clam, conch, mussel, oyster, scallop, and snail, or combinations thereof.

In some embodiments, suitable cells are derived from cephalopods such as cuttlefish, octopus, and squid, or combinations thereof.

In some embodiments, suitable cells are derived from insects such as ants, bees, beetles, butterflies, cockroaches, crickets, damselflies, dragonflies, earwigs, fleas, flies, grasshoppers, mantids, mayflies, moths, silverfish, termites, wasps, or combinations thereof.

In some embodiments, suitable cells are derived from non-arthropod invertebrates (e.g., worms) such as flatworms, tapeworms, flukes, threadworms, roundworms, hookworms, segmented worms (e.g., earthworms, bristle worms, etc.), or combinations thereof.

In some embodiments, the engineered materials (e.g., animal skin, hide, or leather products), include one or more additives in addition to the support material(s). For example, one or more additives are selected from: minerals, fiber, fatty acids, and amino acids. In some embodiments, the engineered animal skin, hide, or leather products, sheet/layers include one or more additives to enhance the commercial appeal (e.g., appearance, color, odor, etc.). In further embodiments, the engineered skin, hide, and leather products, sheet/layers include one or more colorants, and/or one or more odorants.

In some embodiments, the engineered animal skin, hide, or leather products, engineered sheet/layers include one or more of: matrix proteins, proteoglycans, antioxidants, perfluorocarbons, and growth factors. The term "growth factor," as used herein, refers to a protein, a polypeptide, or a complex of polypeptides, including cytokines, that are produced by a cell and which can affect itself and/or a variety of other neighboring or distant cells. Typically growth factors affect the growth and/or differentiation of specific types of cells, either developmentally or in response to a multitude of physiological or environmental stimuli. Some, but not all, growth factors are hormones. Exemplary growth factors are insulin, insulin-like growth factor (IGF), nerve growth factor (NGF), vascular endothelial growth factor (VEGF), keratinocyte growth factor (KGF), fibroblast growth factors (FGFs), including basic FGF (bFGF), platelet-derived growth factors (PDGFs), including PDGF-AA and PDGF-AB, hepatocyte growth factor (HGF), transforming growth factor alpha (TGF-a), transforming growth factor beta (TGF-P), including TGFpi and TGFP3, epidermal growth factor (EGF), granulocyte-macrophage colony-stimulating factor (GM-CSF), granulocyte colony-stimulating factor (G-CSF), interleukin-6 (IL-6), IL-8, and the like.

In some embodiments, the engineered animal skin, hide, or leather products, engineered sheet/layers include one or more preservatives known to the art. In some embodiments, the preservatives are antimicrobial preservatives including, by way of non-limiting examples, calcium propionate, sodium nitrate, sodium nitrite, sulfites (e.g., sulfur dioxide, sodium bisulfite, potassium hydrogen sulfite, etc.) and disodium ethylenediammetetraacetic acid (EDTA). In some embodiments, the preservatives are antioxidant preservatives including, by way of non-limiting examples, butylated hydroxyanisole (BHA) and butylated hydroxytoluene (BHT).

### Engineered animal skin, hide, and leather

Disclosed herein are engineered animal skins, hides, or leather products (e.g., fabricated using these engineered hides). In some embodiments, the engineered animal skin, hide, or leather products are further processed by any known methods in the art. Examples of known methods of processing include processing by preserving, soaking, liming, unhairing, fleshing, splitting, deliming, reliming, bating, degreasing, frizzing, bleaching, pickling, depickling, tanning, thinning, retanning, lubricating, crusting, wetting, sammying, shaving, rechroming, neutralizing, dyeing, fatliquoring, filling, stripping, stuffing, whitening, fixating, setting, drying, conditioning, milling, staking, buffing, finishing, oiling, brushing, padding, impregnating, spraying, roller coating, curtain coating, polishing, plating, embossing, ironing, glazing, and tumbling. It is significant that the methods described herein do not require any of the pre-processing steps that are necessary when using natural animal hide, including de-hairing (unhairing), liming, fleshing, splitting deliming, reliming, etc. The layered bodies formed as described herein may be formed of any appropriate length, and the collagen (and other ECM molecules) formed by the cultured cells, resulting in a layered body that does not include structures such as hair follicles, blood vessels, muscle (e.g., arrector pili muscle), etc.

In general, engineered leather described herein may be tanned (or processed by a similar process) to modify the extracellular matrix material. As discussed above, one of the principle components of the ECM is collagen (and particularly Type I collagen). Tanning may modify the collagen. For example, one tanning agent, chromium(III) sulfate ([Cr(H2O)6]2(SO4)3), has long been regarded as the most efficient and effective tanning agent. Chromium(III) sulfate dissolves to give the hexaaquachromium(III) cation, [Cr(H2O)6]3+, which at higher pH undergoes processes called olation to give polychromium(III) compounds that are active in tanning, being the cross-linking of the collagen subunits. Some ligands include the sulfate anion, the collagen's carboxyl groups, amine groups from the side chains of the amino acids, as well as masking agents. Masking agents are carboxylic acids, such as acetic acid, used to suppress formation of polychromium(III) chains. Masking agents allow the tanner to further increase the pH to increase collagen's reactivity without inhibiting the penetration of the chromium(III) complexes. Collagen's high content of hydroxyproline allows for significant cross-linking by hydrogen bonding within the helical structure. Ionized carboxyl groups (RCO2-) are formed by hydrolysis of the collagen by the action of hydroxide. This conversion may occur during the liming process, before introduction of the tanning agent (chromium salts). The ionized carboxyl groups may coordinate as ligands to the chromium(III) centers of the oxo-hydroxide clusters. Tanning may increase the spacing between protein chains in collagen (e.g., from 10 to 17 Å), consistent with cross-linking by polychromium species, of the sort arising from olation and oxolation. The chromium may be cross-linked to the collagen. Chromium's ability to form such stable bridged bonds explains why it is considered one of the most efficient tanning compounds. Chromium-tanned leather can contain between 4 and 5% of chromium. This efficiency is characterized by its increased hydrothermal stability of the leather, and its resistance to shrinkage in heated water. Other tanning agents may be used to tan the layered body and modify the collagen.

In some embodiments, the engineered animal skin, hide, or leather products are substantially-free of pathogenic microorganisms. In further embodiments, controlled and substantially sterile methods of cell preparation, cell culture, sheet/layer preparation, and engineered animal skin, hide, or leather preparation result in a product substantially-free of pathogenic microorganisms. In further embodiments, an additional advantage of such a product is increased utility and safety.

In some embodiments, the engineered animal skin, hide, or leather products are shaped. In further embodiments, the animal skin, hide, or leather is shaped by, for example, controlling the number, size, and arrangement of the non-contractile sheets/layers used to construct the animal skin, hide, or leather. In other embodiments, the animal skin, hide, or leather is shaped by, for example, cutting, pressing, molding, or stamping. In some embodiments, the shape of the animal skin, hide, or leather product is selected to resemble a traditional animal skin, hide, or leather product.

### Support materials

Any of the engineered materials described herein may include a support material. Support materials may include any exogenous, non-biological material. Although other material may be used, of particular interest are meshes, including sheets of meshes that are woven, extruded or the like, and include openings (e.g., pores) therethrough. In general, embedding the support materials may modify the physical properties of the leather and may also be done in a manner that enhances the ability to manufacture larger sizes (e.g., surface areas) of the material.

For example, an exogenous, non-biological, textured component may be built into or embedded in engineered leather. The support material (or component) may act as a scaffold for the living cells (or a layer of living cells), and the cells/layer of cells may attach to the scaffold and secrete extracellular matrix, thus embedding it completely.

Example of support materials include materials of animal origin (hair, silk, etc.), materials of plant origin (cotton, sheer, sisal, hemp, etc.), materials derived from polymers, petroleum/plastics (e.g., polystyrene, polyester, Kevlar, polyethylene, polypropylene, polyurethane, nylon, etc.) and other polymer materials (e.g., polylactic acid, polyhydroxyalkanoates, etc.), metals, minerals (jewels, rocks, etc.), carbon allotropes (e.g., graphene, nanotubes, fullerenes, etc.), or the like, including combination of these.

In some variations the geometry of the support material may be a substantially two-dimensional (e.g., having a much larger length and width than thickness) layer of material, such as a 2D mesh. The mesh may be created by any appropriate method, including weaving (e.g., knitting, felting, braiding, lacing, etc.), extruding, casting, cutting, or the like. For example, weaving may include weaving with fibers perpendicular to each other or at a range of angles. Other examples of 2D meshes include those created by various methods of etching, perforation, printing, expanding, etc. Meshes may be created by 3D printing, of flat, curved or patterned meshes.

The 2D mesh may be modified to conform to a 3D curved surface (3D composite), or it may be substantially flat. A mesh size and area coverage (including, e.g., the area of holes or strands relative to total area) may vary from micrometers to millimeters. For example, the size of the openings in the mesh may vary from 0 (closed weave) to just <100% (very open weave). Further, the thickness of the 2D mesh may vary, e.g., from atomic dimensions to millimeters. In general, the openings through the mesh may be referred to as the mesh pore size. The mesh pore size ("pore size") may be uniform or variable. As described in more detail below, different mesh pore sizes may interact differently with the cell cultures as described herein.

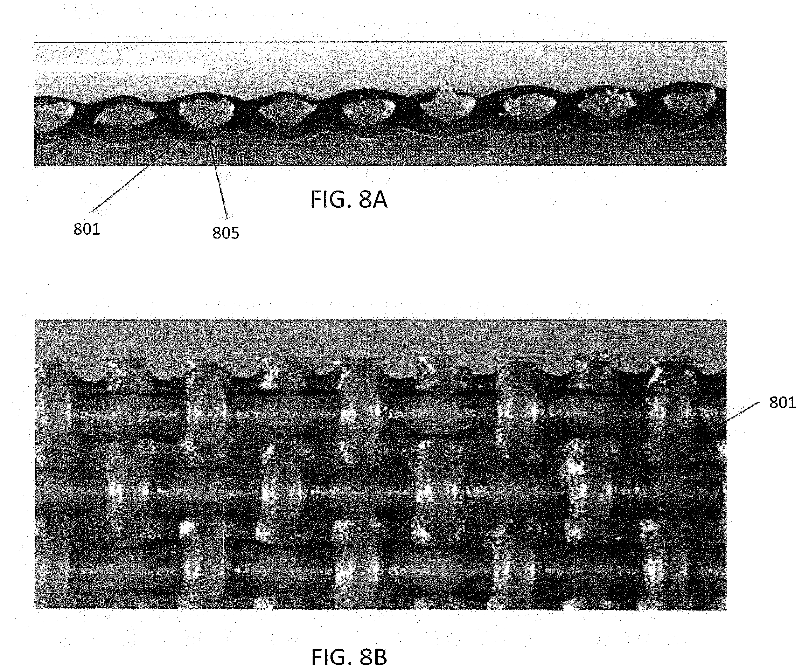

Although other support structures may be used instead of, or in addition to, meshes, there are numerous advantages to using a support structure comprising a mesh. When forming the engineered fabric (e.g., leather), the collagen-releasing cells may bind on both sides of the mesh, and the entire mesh may be easily encapsulated, while remaining flexible and enhancing the durability and other properties. This adhesion may also help in fabricating the mesh. For example, cells may stick to the surfaces of the mesh. Cells may be coated on both sides of the mesh, enveloping the mesh. As illustrated below (e.g., FIG. 8A-8C), the cells forming the engineered hide may be connected by small pieces that grown in the pores or extend between the pores. Although non-mesh (and no-porous) materials may be used, allowing cells to grow on both sides as described herein, there is a benefit to using a mesh having pores allowing fusion of the cells/layers on either side of the mesh. Thus, even meshes that are formed of relatively non-adherent materials, for which cells themselves do not readily attach, may be used as the scaffolding, as the cells/layers of cells used to form the engineered hide may attach through the pores. In general, the material forming the supporting structure may be biocompatible, however, it does not necessary need to provide a surface on which the cells will adhere (or require an intermediary adherent surface). Thus, in general, the mesh may be incorporated or encapsulated (surrounded) into the engineered hide and/or leather. The methods described herein therefore allow a larger range of materials that may be used for forming the composite engineered hide/leather than previously examined.

For example, in some variations the mesh support material (or "mesh") may comprise a flexible net-like structure having a pore size from a few µm to up to 5 mm (e.g., between 2 µm and 3mm, between 2 µm and 1 mm, between 2 µm and 500 nm, etc., or less than about 5 mm, less than about 4 mm, less than about 3 mm, less than about 2 mm, less than about 1 mm, less than about 0.9 mm, less than about 0.8 mm, less than about 0.7 mm, less than about 0.6 mm, less than about 0.5 mm, etc.).

The mesh may be elastic, or fairly rigid. For example, the mesh may have an elasticity that varies from rigid to various elongation values (allowing stretching), and the bending rigidity may from rigid to pliable. In general, the mesh may withstand at least one method of sterilization prior to being added to the sterile cell culture environment (e.g., heat sterilization, steam sterilization, radiation sterilization, etc.). Further, the structure support (e.g., mesh) may be chemically inert, and may withstand chemical treatment(s) without losing structural integrity or material properties, including withstanding the tanning process. For example, the mesh may be able to withstand a wide range of pH in aqueous solution.

Any of the mesh materials described herein may promote cell attachment, either by its inherent surface properties or achieved by surface preparation (e.g., coating, functionalization, etc.). Alternatively or additionally, as mentioned above, the mesh material can be trapped into the leather structure upon assembly without requiring cell attachment to the mesh material itself.

Described below in reference to the figures are examples of methods of incorporating a mesh material within the engineered hides and/or leathers formed from the hides. For examples, FIGS. 1 and 2 schematically illustrate one method of forming an engineered leather that includes the use of a mesh to form a final engineered hide that is reinforced by the mesh material. FIGS. 3-7 illustrate three alternative methods in which cells are cultured on the mesh and layers of collagen (released by the formed layers of cells are formed directly onto the mesh, which may also act as a support during the fabrication process.

In FIG. 1, a tissue explant (e.g., taken from an animal such as a cow) may be extracted and cultured (see, e.g., US-2013-0255003-A1 or P.C.T. application no. PCT/US2014/042384, filed on June 13, 2014). Cells (skin cells) may be extracted through a simple biopsy, and cells then isolated and multiplied in a cell culture medium. In particular, cells may be cultured in a medium as a layer and allowed or inducted to secrete collagen; cells may be spread out so that the collagen forms sheets, as illustrated. The sheets may then treated to prevent contraction, and the thin non-contracting (e.g., decellularized) sheets may then be stacked on top of one another after seeding with additional collagen-forming cells. As part of this procedure, the mesh layer may be added between the layers, and also seeded with cells. This process may result in the formation of thicker sheets of engineered material. In one example, a mesh is placed onto a pre-formed sheet of collagen and cells seeded onto it and allowed to grown and secrete collagen; cells and the secreted collagen, while primarily arranged in the layer over (and at least partially incorporating the mesh, may also attach to either or both the mesh and the layer beneath the mesh. After a suitable time for collagen to be secreted, additional layers may be formed on the top and/or bottom sides of the mesh. For example, one or more additional layers may be formed atop the new layer by seeding with new cells and allowing them to grow and release collagen, and/or additional pre-formed layers of collagen may be added onto the growing (mesh-including) stack, and the new layer allowed to fuse. Additional cells may be added or the cells in the previous layer may act to anchor to the existing collagen layer(s) in the added new layer. Periodically, the stack (including the mesh) may be flipped over so that new layers can be added to the bottom (now top) of the stack. These processes may be repeated as necessary to grow the stack to a desired thickness. Finally, leather is formed from this multilayered structure through a shortened tanning process to modify the collagen.

FIG. 2 schematically illustrates slightly more detail of one variation of the steps described above. In this example thin layers of collagen are formed by culturing in sheets, then the sheets are combined with a mesh material between them, and allowed to fuse. As before, contraction of the cells when cultured in sheets may be prevented by treating the cells (e.g., by killing them) or by other treatments, in which case it may be beneficial to add new cells onto the sheets to enhance fusion of the layers of collagen, as shown in FIG. 2B. Although in theory multiple layers may be added simultaneously, in practice, diffusion may limit the ability of cells to grow when stacked to thicknesses beyond two (or three) layers. FIG. 2C illustrates an example of the planar layer of collagen released by the layers of cells that may be fused together as described.

Alternatively, or additionally, in some variations cells may be directly cultured on and/or around a mesh material to be incorporated into the resulting fabric. For example, FIG. 3A illustrates one example of a mesh material supported in a frame (shown as round, though any shape may be used) which may be used to culture cells to form any of the materials described herein. In this example, the frame may be removed from the mesh, as the mesh will form an integrated part of the resulting fabric (hide/leather). The frame and mesh maybe floated or immersed in a culture medium during growth of the tissue.

FIG. 3B is a schematic illustration of a general method of forming engineered leather materials as described herein. In this example, cells may be first applied (e.g., seeded) on the a first side of the mesh 303. The mesh may be held flat and bathed in a culture medium. In some variations the mesh is held in a frame as shown in FIG. 3A. The mesh may be unsupported (accept for any frame) or it may be backed by a support backing, such as a surface onto which cells may grow and later be removed (e.g., agar, cell culture dish bottom, etc.). Cells may then be cultured on the first (e.g., top) side of the mesh 305, and allowed to grow and release collagen (e.g., in some variation, grow to confluence or near-confluence, as described above). For example, cells may be cultured for hours, days, or weeks (e.g., 1 day, 2 days, 3 days, 4 days, 5 days, 6 days, 7 days, 8 days, 9 days, 10 days, 11 days, 12 days, etc.). Optionally, thereafter, additional cells (and/or a layer of collagen-releasing cells as describe above), may be placed on top of the mesh 307, and allowed to release collagen and further adhere to the growing stack. This step may be sequentially repeated to expand the size of the stack. Thereafter, the mesh may be flipped over 309, and the process repeated on the second (now top) side. Thus, additional cells (and/or layers of collagen or cells and collagen) may be placed on the second side 313 and cultured and allowed to release additional collagen to adhere 315. As before additional cells and/or layers of collagen (or collagen and cells) may be placed onto this second side and cultured to expand the thickness of the material 317. The material may again be flipped over and the steps repeated to continue to grow the material. Once the desired thickness is reached, the process may be stopped, and the material, now with embedded mesh, may be removed and treated (e.g., tanned).

Alternatively, in some variations, multiple layers/stacks with an embedded mesh may also be stacked onto each other to form thicker layers. For example, a mesh may be grown with one or more layers as described in FIG. 3B, and an additional layer of mesh may also be stacked onto the growing material, and incorporated therein by adding additional cells (which then secrete collagen) onto the growing stack. Thus, multiple mesh layers may be incorporated into a single material, enhancing the material properties.

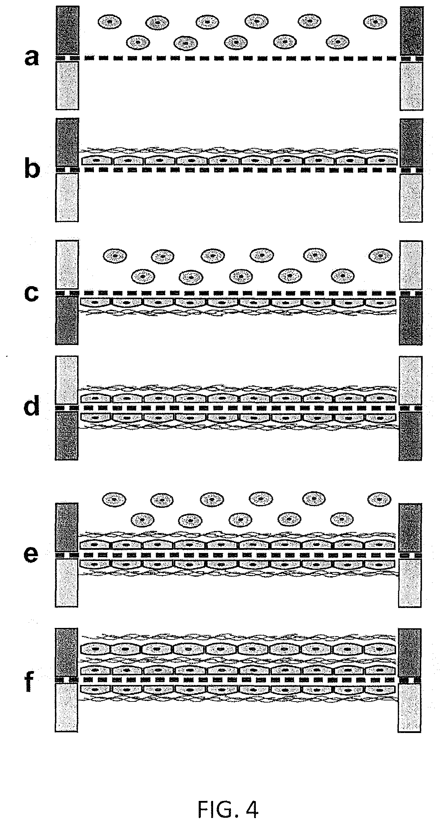

FIG. 4 illustrates one variation of a method as described above. In FIG. 4, a relatively small mesh size is shown (e.g., pore size of between 2-300 µm). Small mesh sizes may be capable of trapping cells or cellular aggregates (e.g., typical cell size 10-20 micrometers, typical aggregate size of 100-500 micrometers). As mentioned above, the mesh may be mounted into a frame (e.g., circular, rectangular, free-form 2D frame) that immobilizes and straightens the mesh, creating a smooth, planar and horizontal surface, as illustrated in FIG. 3A. The framed mesh may be transferred into a container (tissue culture Petri dish) containing tissue culture medium, then seeded with cells as shown in FIG. 4a. The cells may then attach, spread and secrete extracellular matrix (collagen) on the top side of the mesh (FIG. 4b). After a predetermined period of time (in which the cells release collagen to form a layer of collagen) the frame may be inverted and the other side is seeded with cells, as shown in FIG. 4c. In FIG. 4b and 4c collagen may also extend between the pores (not shown). The new cells may also attach and secrete a layer of collagen on the other side of the mesh (FIG. 4d). This procedure can be iterated (FIGS. 4e and 4f) to achieve the desired thickness of the engineered material. The resulting material, with the embedded mesh, may be subsequently tanned. Note that this figure is not to scale.

In this example, the small mesh sizes may allow the cells (or clumps of cells) to sit directly on the mesh, and collagen is still released between the pores (not shown) fusing the layers on both sizes of the mesh, and locking the mesh in position. Thus, even with materials that cells don't typically adhere to may be encapsulated as shown.

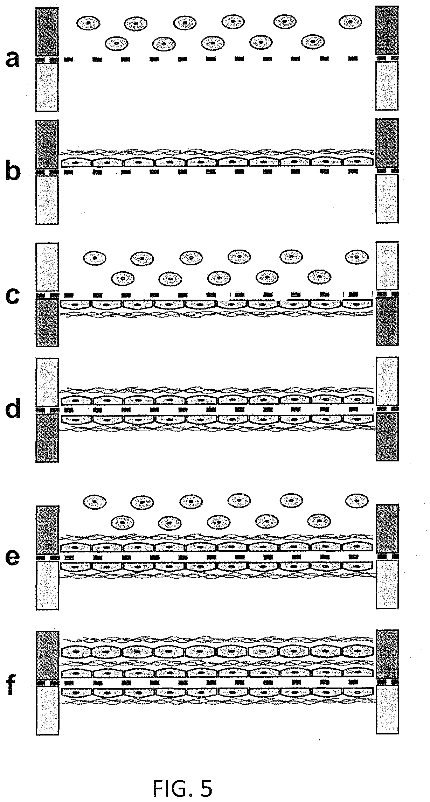

For slightly larger pore sized meshes (e.g., meshes having a pore size larger than 300 micrometers), cells may also be grown directly onto the mesh, as shown in FIG. 5. In FIG. 5a, cells are seeded into a tissue culture dish containing culture medium and cultured for a predetermined time period, during which the cells secrete a layer of extracellular matrix (in particular, collagen) as shown schematically in FIG. 5b. The unrestrained mesh is placed then on this cell-extracellular matrix layer and seeded again with cells (FIG. 5c). The number of cells in the second seeding may vary depending on the thickness and area coverage of the mesh. During the second incubation time interval (which may be the same as the first) the cells from the second seeding will divide and eventually fill the holes of the mesh while secreting extracellular matrix and coupling the mesh and the underlying cell/extracellular matrix layer as shown in FIG. 5d. The seeding procedure may be repeated again (FIG. 5e) to completely cover the mesh, creating a top layer of ECM, cultured for the same duration as for the previous two layers (FIG. 5f). The mesh-ECM sheet composite may then detached from the dish and cultured in a nonadhesive environment until ready for tanning, or additional layers may be made to increase thickness. For example, the mesh/ECM composite can be restrained into the frame and further seeded regularly inverting the frames to achieve a coating of the same thickness on both sides.

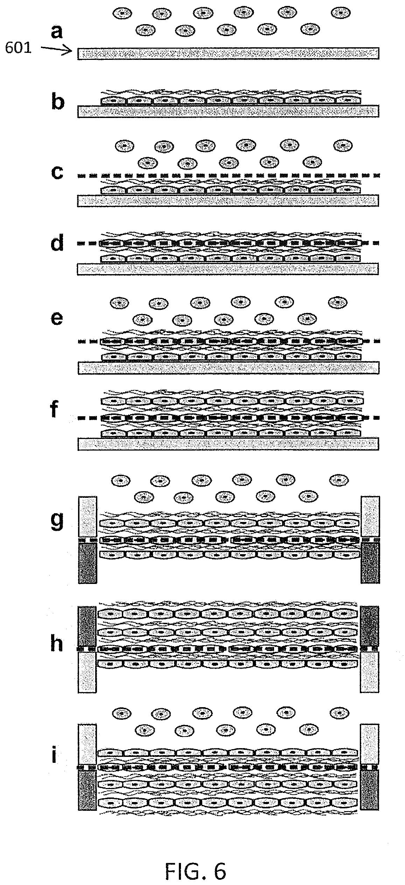

Another example (also not shown to scale) is illustrated in FIG. 6. In this example, a large pore size (e.g., greater than 500 µm (e.g., between 0.5 mm and 5 mm) is used. In FIG. 6, the mesh is placed onto a culture dish or substrate 601 (potentially including, as mentioned above, onto an existing layer of collagen, e.g., decellularized layer). In this example, cells are cultured first directly onto the substrate (FIG. 6a) and allowed to release collagen (FIG. 6b). A mesh (large pore size) is then applied as shown in FIG. 6c. Cells (and/or an additional layer) are then applied onto and in the mesh, as shown in FIG. 6c and 6d, and cells allowed to grown and release collagen. Thereafter, additional layers may be added by sequentially adding cells and culturing them to grow, as illustrated in FIG. 6e-6h. The stack including the mesh may then be removed from the plate (detaching the entire thing from the dish, as cells stick to the mesh better than the surface of the dish), and the mesh flipped over, and additional cells/layers applied, as shown in FIG. 6i.

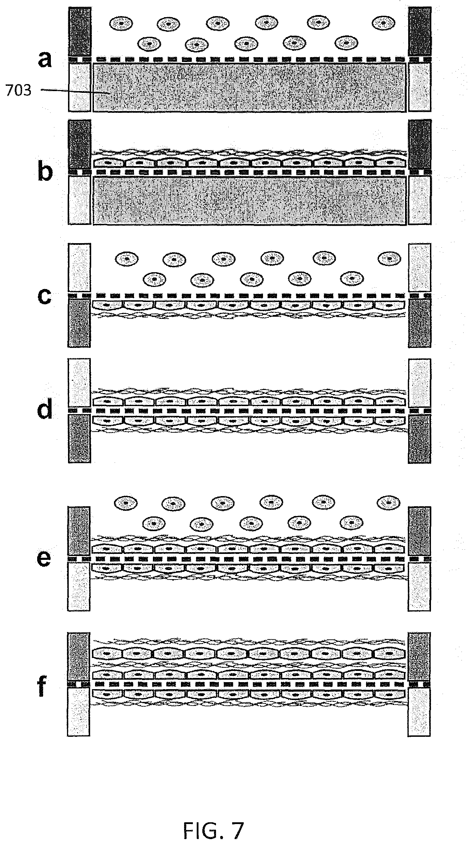

Another variation using a large pore-size mesh is shown in FIG. 7. When the interaction between the cells and the culture dish may be stronger than the interaction between the cells and the mesh, detaching the composite structure from the culture dish (FIG. 6f.) might cause the cell/ECM layer to peel off the mesh, which is undesirable. Thus, in some variations the mesh may be restrained in a frame and a nonadhesive (e.g. Teflon, agarose, etc.) plug 703 may be placed below the mesh, and in contact with it. The mesh may then seeded with cells (FIG. 7a), where cells will either accumulate in the openings and on the top surface of the mesh, secreting extracellular matrix (FIG. 7b). The plug may then be removed, and the mesh inverted and seeded with cells on its other side (FIG. 7c). Sequential inversion, seeding and cell culture may then be performed to deposit multiple layers on both sides to thicken the construct (FIGS. 7c, 7d, 7e, 7f).

Any of the composite mesh/ECM constructs described herein can be tanned either before or after releasing them from the frame, or tanning it while restrained. Tanning while restrained by the frame may be desirable when the tanning agents are excessively astringent and would deform the construct. Thus, a releasable frame may be used. and released after tanning.

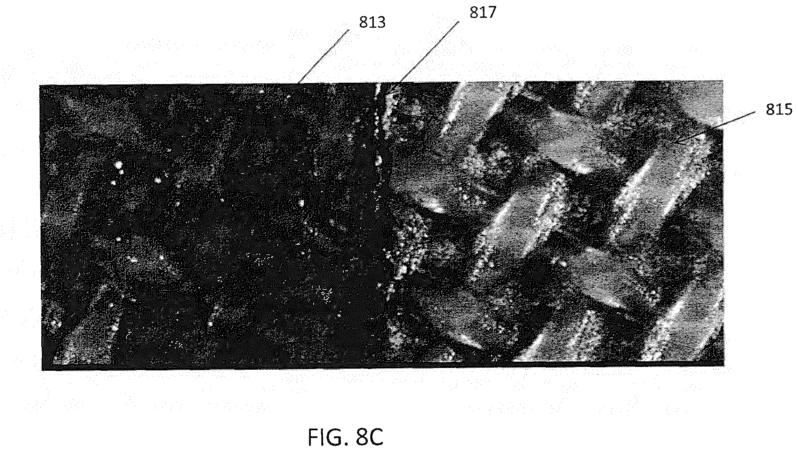

A proof of concept experiment is shown in FIGS. 8A-8C. In this example, a polyester-leather composite was prepared with the method illustrated in FIG. 6, above. After detaching the structure from the culture dish, the composite was further cultured for 3 weeks in a nonadhesive environment, subsequently tanned using standard chromium tanning methods. Even after tanning, the collagenous material completely covers the mesh with a thin layer of engineered leather, as shown in FIG. 8A and 8B. The polyester mesh 801 is encapsulated with tanned collagen 805. A cross section shows even coverage on the threads 801 (FIG. 8A), complete filling the mesh (FIG. 8B). In FIG. 8C, the resulting material was deconstructed by partially removing the top layer 813 prior to tanning (e.g., when removing from the plate) to illustrate the triple layered structure. The underlying mesh 815 is exposed to the right of the torn 817 region.

Cells in the emerging sheets formed as described above may secrete and assemble extracellular matrix. For each seeding, the rate of secretion and assembly may depend on cell type and slows down after a certain thickness of the sheet is attained. This may result from either (i) the cells exhausted their capacity of producing extracellular matrix, (ii) the layer of extracellular matrix prevents the nutrients in the culture medium to reach the cells, or (iii) the matrix is degraded or remodeled by the cell's enzymatic activity.

In response, the sheets being formed (e.g., layer of cells and extracellular matrix) may be seeded with fresh collagen-releasing cells either of the same or different type as in the already formed layer. These new cells typically attach strongly to the collagen in the underlying layer and start dividing and producing new extracellular matrix. The above reseeding procedure can be iterated (i.e. repeated) until the desired thickness of the emerging multilayered tissue construct is achieved.

In general, during culture time, both the newly added cells as well as the cells in the initial layer may secrete collagen, resulting in the interconnection between the collagen layers. Secretion by the cells in the initial layer may occur at a slower rate than by the newly added cells as they are getting farther from the culture medium on both sides of the mesh.

In some variations two reseedings are performed leading to a three-layered structure on both sides of the mesh. In other variations the number of reseedings may be larger than 3. Note that with the increase of the number of reseedings the cells in the lower layers become progressively farther away from the interface of the construct with the nutrients-containing culture medium. This may lead to the slowing down of their cell functions, in particular the release of collagen, and eventually may lead to their death by starvation.

In some variations the samples are further coated with a polymer to impart further functionality on the material, e.g. waterproof, very high strength, chemical resistance, etc.

In general, each layer is formed by culturing the cells until a layer of collagen is formed within the new layer. This may take, for example, about a week (e.g., between about 5-10 days). For example, if a layer needs 10 days to grow, in some variations, each side may be re-seeded before the layer is completely grown, and the structure maybe flipped after cells are sufficiently adherent (within a few days).

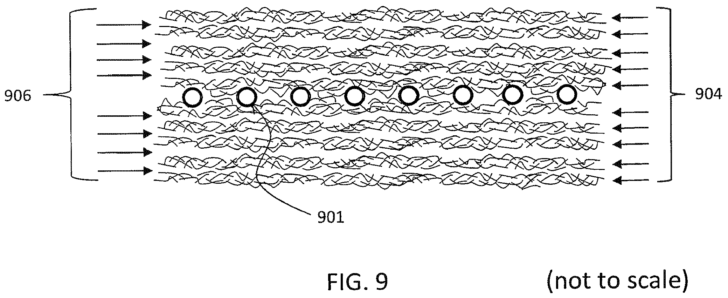

Any of the engineered materials (fabrics, hides, leathers, etc.) described herein may have a distinct ultrastructure that includes the embedded material (e.g., mesh), but is also layered, as a result of the method of fabrication described. For example, FIG. 9 schematically illustrates an ultrastructure of an engineered material having multiple layers in which a mesh material is embedded. In FIG. 9, the mesh 901 is embedded within a plurality of layers formed by any of the methods described herein. The result is layers in which a cross-section shows strata of collagen dense 904 regions alternating with less-dense regions 906. The less-dense regions may correspond to the cellular region, and the region of adhesion between the sequentialy applied layers. This ultrastructure is unlike native materials, both in the inclusion of the mesh material 901 and in the layered formation of the collagen.

The methods described herein represent a very flexible method for embedding virtually any support material (e.g., mesh) into an engineered leather, which may substantially change the physical properties of the leather. Although the examples described herein are specific to meshes, it should be understood that non-mesh materials (e.g., strands, etc.) may also be used.

### Fibrous scaffolds

Also described herein are engineered leathers formed using a support comprising a fibrous scaffold that is tanned (e.g., cross-linked) to the cultured cells and/or any extracellular matrix (ECM) components released by the cultured cells to form. The resulting engineered leather may be referred to as a fiber-reinforced tissue composite and may have superior properties (e.g., durability, strength, etc.) compared to other engineered leathers. Also described are methods of forming these fiber-reinforced tissue composites, including methods of growing/culturing them and methods of tanning them.

In general, the scaffolds described herein are configured to be cross-linked to the released extracellular matrix (e.g., collagen) from cells cultured on the scaffold. These scaffolds may be formed of a material capable of forming cross-links with ECM and/or the cells during tanning. Further, the structure of the scaffold (e.g., porosity, fiber length, fiber density, etc.) may be chosen to permit cross-linking and/or to encourage growth of the cells and release of extracellular matrix. The scaffold is tanned with and to the ECM to form the final product, the fiber-reinforced tissue composite. Thus, the scaffold forms an integral part of the final product, and its dimensions, including the thickness, may help determine the final thickness of the resulting leather.

One particular example of a fibrous scaffold that may be used as described herein is silk. Silk (e.g., organic and/or synthetic silk) may be formed (e.g., spun) to a predetermine fiber thickness and used in a woven and/or non-woven sheet forming the scaffold onto which ECM-releasing cells may be cultured. Such cells may be, e.g., dermal fibroblasts, smooth muscle cells, etc.

In general, the scaffold is formed of a material (such as silk) that can be tanned to cross-link reactive groups on the scaffold (e.g., amine and carboxylic acid groups) with the released ECM (e.g., collagen).

The fibrous scaffold may provide a great deal of surface area onto which cells may be cultured. For example, the scaffold may be formed of relatively dispersed fibers forming a dispersed fibrous scaffold. A fibrous scaffold may have a lot of surface area to allow cell growth and to allow for a strong fiber-tissue matrix interface (which may also enhance overall strength of the resulting composite). In some variations, the length of the fibers forming the scaffold is between 100 nm and 100 µm. In some variations, the length of the fibers is between 100 nm and up to 1 mm, 10 mm, 100 mm or 1 m. The density of the fibrous material in the scaffold may be between 10 and 100 mg/cc. In some variations the density of the fibrous material is between 10 and 10,000 mg/cc. The porosity of the fibrous scaffold (including of the woven/non-woven fibers) may be between 10 and 99%.

In general, it may be advantageous to provide a high porosity. This may permit a lot of cell infiltration and tissue growth, including within the thickness of the scaffold. The scaffold may be any appropriate thickness (e.g., between 10 µm and 5 mm, e.g., between 100 µm and 1 mm, between 100 µm and 500 µm, between 50 µm and 300 µm, etc., or between any lower value of 10 µm, 30 µm, 50 µm, 75 um, 100 um, 200 um, 300 µm , etc. and an upper value of 50 µm , 100 µm, 150 µm, 200 µm, 300 µm, 400 µm, 500 µm, 600 µm, 700 µm, 800 µm, 900 µm, 1 mm, 2 mm, 3 mm, 4 mm, 5 mm, etc.). The porosity may be determined as the space (e.g., average diameter) between fibers.

In operation, the method of forming the fiber-reinforced tissue composites described herein may include culturing cells on the fibrous scaffold until a desired amount of ECM (e.g., collagen) has been formed on and/or over the fibrous scaffold. Optionally, the fibrous scaffold may be formed and/or prepared to receive the cells to be cultured thereon. For example, the scaffold may be treated to expose reactive groups or cell-attachment sites. In some variations the fibrous scaffold may be applied in a cell reactor for seeding and growth of the cells on the scaffold. As an example, cells, which may be, for example, a mammalian cell line having known ECM releasing properties, be seeded onto the prepared fibrous scaffold and allowed to grow, divide and set down ECM. Cells may be cultured in typical culture conditions for an appropriate length of time (e.g., 2-90 days). Additional cells may be added during this period. Thereafter, the resulting material, comprised of the fibrous scaffold with released ECM and cells, may be referred to as an intermediate (untanned) tissue construct, which may be further processed. This intermediate (untanned) tissue construct may be decelluarlized (e.g., by treatment with alcohols or other agents) or not. The intermediate (untanned) tissue construct may then be processed by tanning, which may include traditional tanning chemistries as well as crosslinking reactions to reinforce the scaffold to the tissue.

Provided below is an example of various reinforcement and tanning methods that may be used and/or modified. The fibrous scaffold and the cultured ECM grown on the scaffold are tanned (and cross-linked) together by this process, and the resulting fiber-reinforced tissue composites may be used as an engineered leather material.

A reinforcement step may be included to crosslink the deposited ECM to the fibrous scaffold encased in ECM, followed by a tanning procedure to produce the look and feel of traditional leather. While traditional tanning chemistries may effectively crosslink ECM to scaffold if the scaffold has proper functionality (such as surface amine and carboxylic acid groups), other scaffold surface chemistries and cross linkers may be incorporated to strengthen the ECM-scaffold interface. These include epoxide, acrylate, aldehyde, sulfhydryl, diazirines, aryl-azides, etc., as well as protected chemistries that may be activated following tissue growth to reduce cytotoxicity of the scaffold surface. The scaffold may be modified with reactive chemistries prior to cell seeding that are pendant from spacer molecules of size between 0 Da and 100MDa. The scaffold surface chemistry may be reacted directly to the ECM or through cross linkers with functionality between 1 and 2000 and with size between IDa and 100MDa. Further, crosslinking molecules may be polymerized within the scaffold-ECM construct with a size between IDa and 100MDa. Polymerization of these cross linkers may be initiated following tissue growth with triggers such as light exposure, temperature change, addition of chemical initiators, etc.

In general, any appropriate tanning methods may be used, including method derived from traditional tanning, which may result in leather having the look and feel of traditional tanned hides. For example, following culturing of cells on the scaffolds as described above (e.g., in a sterile environment), the intermediate (untanned) tissue construct consisting of cells and ECM grown on the planar (e.g., relatively thin, but long and wide sheet of) fibrous scaffold may be washed (e.g., to remove culture medium), and tanned. Traditional steps such as liming (e.g., treatment with a basic compound such as milk of lime and/or the addition of "sharpening agents" including disulfide reducing agents such as sodium sulfide, cyanides, amines, etc.) may be included. Such steps may be modified, however, as the intermediate construct does not include hair, nail and other keratinous matter typically found in native skins. Such steps may be maintained or modified to remove some ECM materials and/or to swell and split fibers in the intermediate construct or otherwise prepare collagen in the construct for tanning.

The methods may include or avoid (as unnecessary) the use of unhairing agents such as sodium sulfide, sodium hydroxide, sodium hydrosulfite, calcium hydrosulfide, dimethyl amine, and sodium sulfhydrate.

A step that removes cellular material while preserving the ECM may also be included. Decellularization methods used in tissue engineering may be used for this purpose including the use of surfactants, enzymes, ultrasonic energy, freeze thaw cycles, etc.

A deliming step may also be included. For example, the pH of the collagen may be brought down to a lower level so that enzymes may act on it. Depending on the end use of the leather, the intermediate construct may be treated with enzymes to soften them, a process called bating. If bating is used, once completed, the intermediate tissue construct may be treated first with salt and then with sulfuric acid, if a mineral tanning is to be done, which may bring down the pH of collagen to a very low level so as to facilitate the penetration of mineral tanning agent into the substance. This process is known as pickling. The salt (e.g., sodium chloride) may penetrate faster than the acid and limit the ill effect of sudden drop of pH. If vegetal tanning is used, the tanning agent may be tannin. The tannins are a class of polyphenol astringent chemicals that occur naturally in the bark and leaves of many plants. Tannins bind to the collagen proteins and may hide and coat them, causing them to become less water-soluble and more resistant to bacterial attack. The process may also cause the material to become more flexible. Traditionally, primary barks, processed in bark mills and used in modern times, are chestnut, oak, redoul, tanoak, hemlock, quebracho, mangrove, wattle, and myrobalans. Traditionally, hides are stretched on frames and immersed for several weeks in vats of increasing concentrations of tannin. The intermediate tissue constructs described herein may provide easier, more direct access to the tannins and may therefore require less processing time in general.

In chrome tanning, prior to the introduction of the basic chromium species, several steps may be used to prepare the material, as mentioned above, including the introduction of alkali agents such as sodium hydroxide, restoring neutral pH, bating (softening with enzymes), and pickling (lowering pH of the material being processed, e.g., with salt and sulfuric acid).

In traditional tanning, the pH is very acidic when the chromium is introduced, to ensure that the chromium complexes are small enough to fit in between the fibers and residues of the collagen. Once the desired level of penetration of chrome into the substance is achieved, the pH of the material is raised again to facilitate the process. This step is known as basification. Chrome tanning is typically faster than vegetable tanning.

Chromium(III) sulfate ([Cr(H₂O)₆]₂(SO₄)₃) has long been regarded as the most efficient and effective tanning agent. Chromium(III) compounds of the sort used in tanning are significantly less toxic than hexavalent chromium. Chromium(III) sulfate dissolves to give the hexaaquachromium(III) cation, [Cr(H₂O)₆]³⁺, which at higher pH undergoes processes called olation to give polychromium(III) compounds that are active in tanning, being the cross-linking of the collagen subunits. The chemistry of [Cr(H₂O)₆]³⁺ is complex due to the presence of a variety of ligands. Some ligands include the sulfate anion, the collagen's carboxyl groups, amine groups from the side chains of the amino acids, and masking agents. Masking agents are carboxylic acids, such as acetic acid, used to suppress formation of polychromium(III) chains. Masking agents allow the tanner to further increase the pH to increase collagen's reactivity without inhibiting the penetration of the chromium(III) complexes.

As mentioned above, collagen is characterized by a high content of glycine, proline, and hydroxyproline, usually in the repeat -gly-pro-hypro-gly-. These residues give rise to collagen's helical structure. Collagen's high content of hydroxyproline allows for significant cross-linking by hydrogen bonding within the helical structure. Ionized carboxyl groups (RCO2-) are formed by hydrolysis of the collagen by the action of hydroxide. This conversion occurs during the liming process, before introduction of the tanning agent (chromium salts). The ionized carboxyl groups coordinate as ligands to the chromium(III) centers of the oxo-hydroxide clusters.

Tanning increases the spacing between protein chains in collagen from 10 to 17 Å. The difference is consistent with cross-linking by polychromium species, of the sort arising from olation and oxolation.

Subsequent to application of the chromium agent, the bath may be treated with sodium bicarbonate to increase the pH to 4.0-4.3. This increase induces cross-linking between the chromium and the collagen. The pH increase may normally be accompanied by a gradual temperature increase up to 40°C. Chromium-tanned leather can contain between 4 and 5% of chromium. This efficiency is characterized by its increased hydrothermal stability of the leather, and its resistance to shrinkage in heated water.

Other forms of tanning may be used, including ones based on alum, zirconium, titanium, iron salts, or a combination thereof. Tawing may be used on the intermediate tissue constructs described herein. Tawing is a method that uses alum and aluminum salts, generally in conjunction with other products such as egg yolk, flour, and other salts. The material becomes tawed by soaking in a warm potash alum and salts solution (or equivalent), between 20 and 30°C. The process may increase the resulting leather's pliability, stretchability, softness, and quality. Adding egg yolk and flour (or equivalents) to the standard soaking solution may further enhance its fine handling characteristics. Then, the intermediate tissue construct is dried and allowed to stabilize.

Depending on the finish desired, the material may be waxed, rolled, lubricated, oiled (e.g., injected with oil), and/or dyed. Suedes, nubucks, etc. may be formed, e.g., by inducing surface finishes. The material may be additionally finished by retawing. Retawing agents and/or dyes may be applied to the material to enhance the physical strength and properties desired depending on the end product. A final stage, finishing, may be used to apply finishing material to the surface or finish the surface.

### Examples

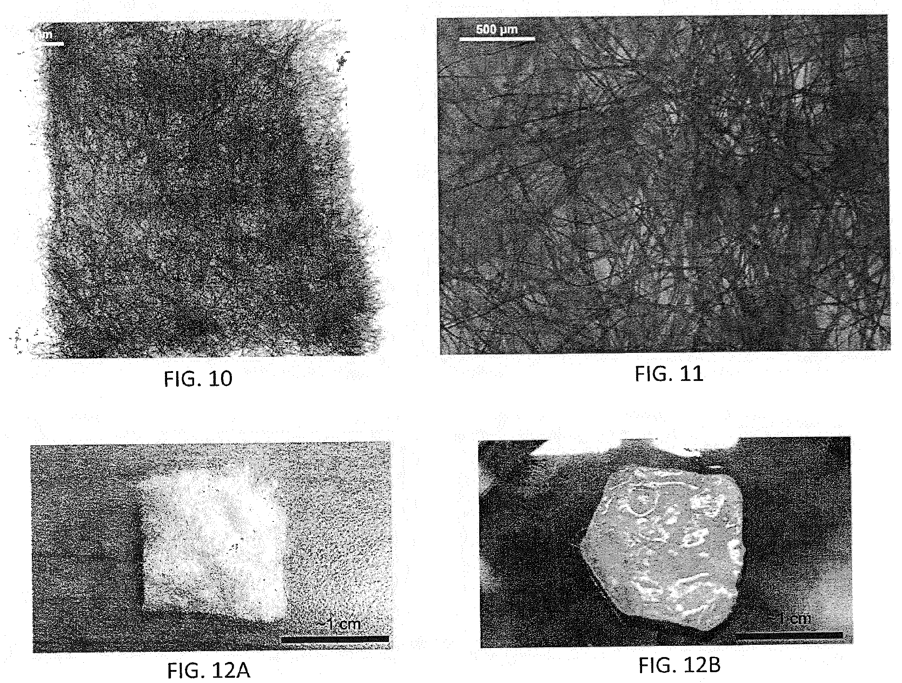

FIGS. 10-16 illustrate example of fiber-reinforced tissue composites and methods of forming them as described above.

For example, FIGS. 10 and 11 show an example of a fibrous tissue scaffold that may be used. In this example, the scaffold is formed of silk fibers. The scaffold has a thickness of about 0.5 mm, and fiber diameters of about 15-20 microns (e.g., on average/mean). In this example, the overall material density is approximately 80 mg/cc, which corresponds to a porosity of about 95%. The orientation of fibers may affect overall materials properties, and the assembly process may be varied to produce a variety of non-woven, woven and knit architectures. In addition, the length of the fibers may effect overall material properties, and the fiber length, diameter and/or porosity may be varied. For example, nanofibers may be used having from about 10 µm (microns) to 20 µm or more. FIG. 11 shows an enlarged view of a region of the scaffold shown in FIG. 10.

FIG. 12A shows an example of a "dry" fibrous (silk) scaffold prior to seeding with cells. In FIG. 12B, the scaffold shown in FIG. 12A has been seeded with cells (in this example, bovine dermal fibroblasts) at density of 8.5×10⁶ cells/ml. The volume of the scaffold was 100 µL/cm² of scaffold (similar to that shown in FIG. 10-11). In this example, cells are seeded on top of the scaffold and have been seen to settle (e.g., via gravity) onto the porous scaffold. Thereafter, the scaffold can be turned, flipped, rotated, etc., which may help distribute the cells and/or culture medium throughout the substrate. In practice it may be beneficial to have a fibrous substrate that is initially greater than 50% porous. As mentioned above, in some variations, the scaffold may be modified to enhance cell adhesion.

In the example, shown in FIG. 12B, the cells may be cultured for approximately 4 weeks, with regular change in media (e.g., every few days); standard tissue culture techniques may be adapted for use with the scaffold; for example, growth factors may be used, or agents to enhance the release and/or type of ECM deposited. In addition, dynamic culture environments such as perfusion or mechanical loading may be used to enhance ECM deposition. In FIG. 12A-12B, the scaffold has a thickness of about 0.5 mm. Thicker scaffolding may also be used (e.g., up 5 cm). After four weeks of culture the scaffold fibers are surrounded by tissue (including collagen).

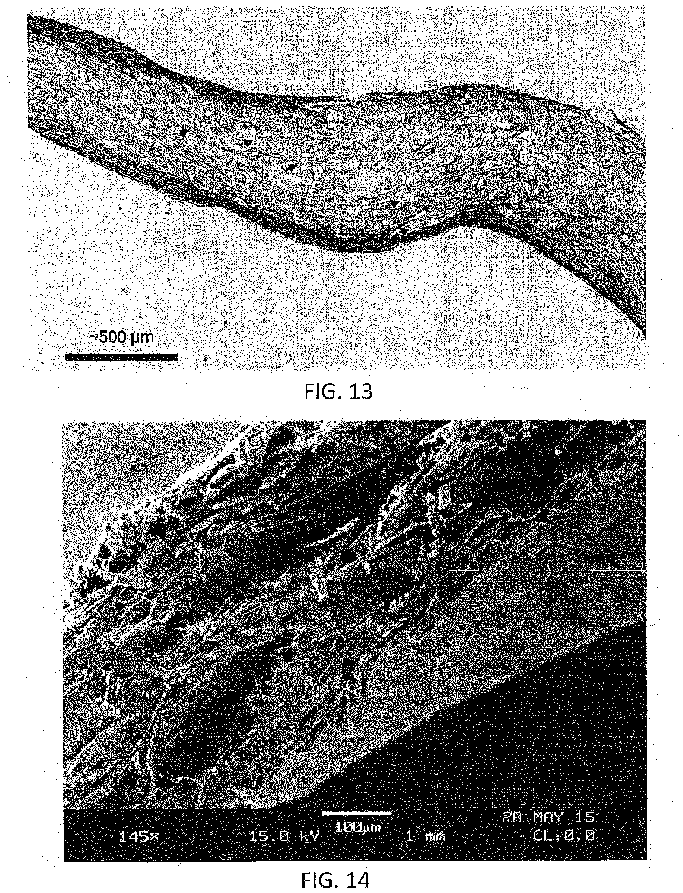

FIG. 13 shows a section taken from the exemplary intermediate (untanned) tissue construct shown in FIG. 12B in cross-section through the tissue. In this example the stain shows (red in original color version) collagen; scaffold fibers are indicated (in section) by the arrowheads. The sample is shown having a dense network of collagen extending around and through (e.g., within the porous fibrous scaffold). This sample was fixed for histology. A scanning electron microscopic image of an intermediate (untanned) tissue construct similar to that shown in FIG. 12B is provided in FIG. 14.

In FIG. 14, a section through a fibrous scaffold onto which cells releasing collagen have been cultured is shown. The fibrous scaffold in this example is silk, and the SEM shows collagen-rich tissue grown throughout the silk fiber scaffold.

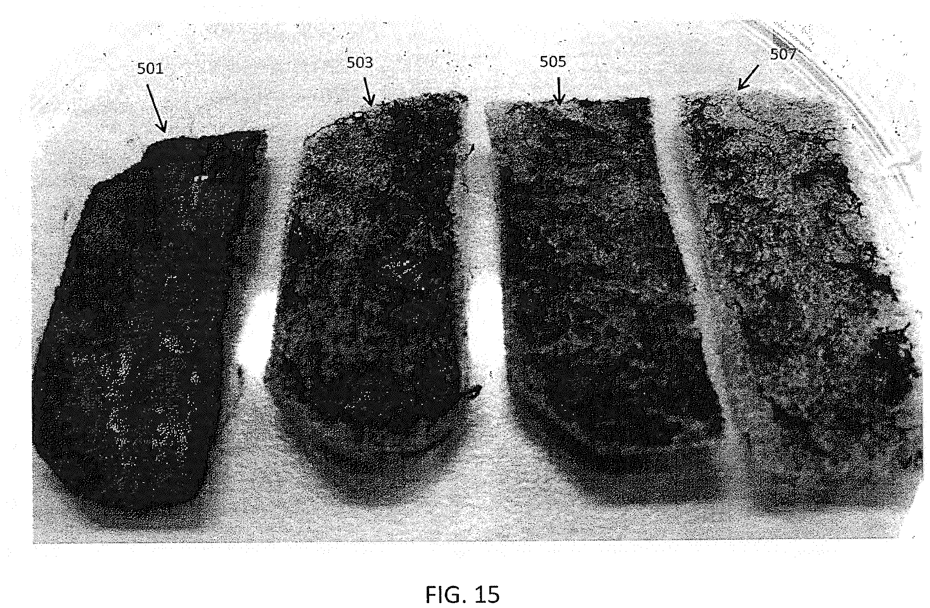

FIG. 15 shows a comparison between fibrous scaffolding that does (e.g., silk) and does not (e.g., polylactic acid, PLLA) allow reinforcement crosslinking during a tanning process as described herein. In FIG. 15, cells have been cultured equivalently on four different fibrous scaffolds and tanned: silk 501, high density PLLA 503, polyester 505, and low density PLLA 507. Only the silk scaffold included amine and carboxylic acid groups which crosslink with the tissue/ECM during the tanning process (chromium salt tanning). Each of these scaffolds are otherwise approximately equivalent. Each are about 4 cm long and about 1.5 cm wide; the silk 501 and low density PLLA 507 are each 0.5 mm thick and the high density PLLA and polyester are each about 1 mm thick. Following tanning, only samples in which the scaffold was cross-linkable when tanning (e.g., silk 501) resulted in materials having a texture and look similar to native leather. Tanned silk without cells cultured on it does not; the resulting material (silk alone) pulls apart.

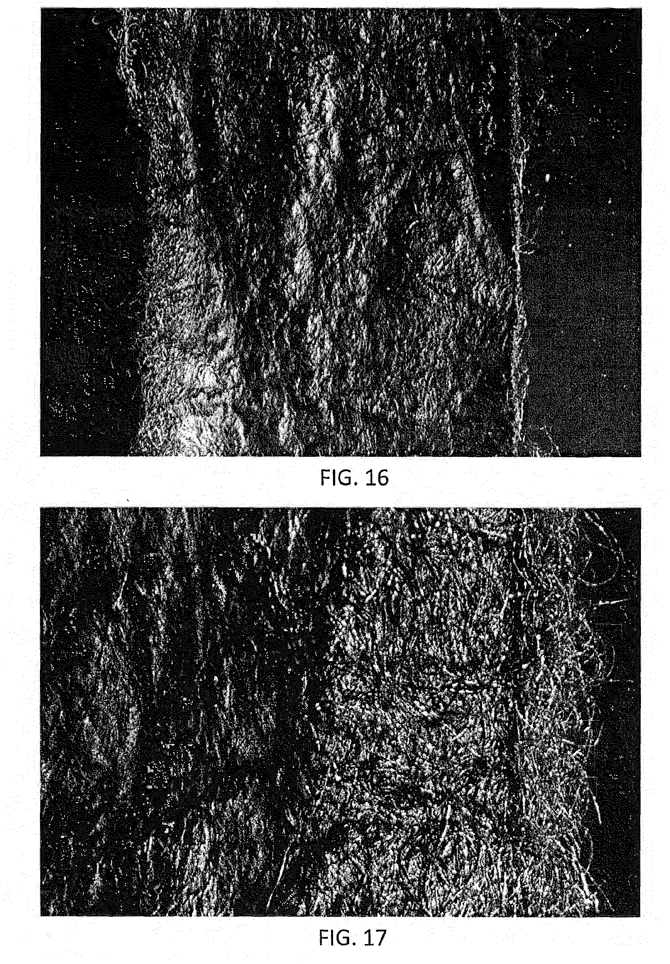

FIGS. 16 and 17 illustrate example of fiber-reinforced tissue composites formed as described herein after tanning. FIG. 16 shows an outer surface of a tanned material that has been formed and processed as described above. In this example, the leather-like outer surface feels similar to native leather. The material is formed of a silk fibrous scaffold onto which cells (dermal fibroblasts) have been cultured, and the resulting intermediate tissue construct has been tanned. FIG. 17 shows an enlarged view of an edge region of a tanned (silk) fiber-reinforced tissue construct. After tanning, a gradient of tissue was produced toward the edge of the silk scaffold, revealing the silk fibers dispersed through the tissue matrix (on the right in the figure).

In general, the resulting product will have a look and feel similar to native leather, but is detectably (e.g., under examination of the ultrastructure) different. For example, the resulting material will have the fibrous scaffold material dispersed throughout, and typically surrounded by ECM (e.g., collagen); the collagen and the scaffold are both tanned and cross-linked together. There will typically be an outer layer of tissue on the scaffold.

For example, described herein are methods of forming a fiber-reinforced biological tissue composite, the method comprising: culturing cells on a fibrous scaffold until fibers of the fibrous scaffold are surrounded by tissue comprising extracellular matrix, forming an intermediate tissue construct; and tanning the intermediate tissue construct to cross-link the fibers of the fibrous scaffold to the tissue.

The culturing may comprise culturing cells on a fibrous silk scaffold, culturing cells on a fibrous protein scaffold, etc. The fibrous protein scaffold may comprise one or more of: a fibrous wool scaffold, a fibrous silk scaffold or a fibrous collagen scaffold.

Culturing may comprise culturing cells on a protein fiber scaffold comprising amine, carboxylic acid and sulfhydryl groups. Culturing may comprise culturing cells on a fibrous cellulosic scaffold. The fibrous cellulosic scaffold may comprise one or more of a fibrous flax scaffold, a fibrous pineapple scaffold, or a fibrous cotton scaffold. Culturing may comprise culturing cells on a synthetic fiber scaffold with a cross linkable surface chemistry.

The fibrous synthetic scaffold may comprise one or more of a carbon fiber scaffold, a Kevlar scaffold or a fiberglass scaffold.

Culturing may include culturing the cells on the fibrous scaffold until the fibers are surrounded by collagen. Culturing may include culturing cells on the fibrous scaffold comprising fibers having a length of between about 100 nm and 1 m. Culturing may include culturing cells on the fibrous scaffold having a porosity of between 10 and 99%. Culturing may comprise culturing mammalian cells on the fibrous scaffold.

Tanning may include crosslinking amine, hydroxyl, sulfhydryl, tyrosyl, and carboxylic acid groups on the extracellular matrix proteins in the tissue to the scaffold. Tanning may comprise crosslinking the fibers of the fibrous scaffold to the tissue through covalent bonds. Tanning may comprise crosslinking the fibers of the fibrous scaffold to the tissue through ionic, hydrophobic and van der Waals interactions.

A fiber-reinforced biological tissue composite material may include: a fibrous scaffold comprising a plurality of fibers, wherein the fibers are surrounded by extracellular matrix, wherein the extracellular matrix and plurality of fibers are cross-linked to each other.

The scaffold may be a naturally occurring protein fiber containing amine hydroxyl, sulfhydryl, tyrosyl, and carboxylic acid groups. The scaffold may be a naturally occurring cellulosic fiber containing amine, hydroxyl and carboxylic acid groups. The scaffold may be a synthetic fiber containing surface chemistries to crosslink to the ECM. The fibers may be chemically modified to enhance tissue crosslinking to scaffold.

The scaffold may be chemically modified to contain tissue crosslinking groups including one or more of: amines, carboxylic acids, sulfates, aldehydes, hydrazides, sulfhydryls, diazirines, aryl-azides, acrylates and epoxides.

The tissue crosslinking groups may be protected during tissue growth and activated for crosslinking when tissue growth is complete. The tissue crosslinking groups may be pendant to the scaffold with a spacer between 1 dalton and 100 megadaltons.

The scaffold may be crosslinked to the tissue through non-covalent interactions including ionic, hydrophobic and van der Waals interactions. The scaffold may be crosslinked to the tissue through covalent bonds. The scaffold may be directly reacted with amine, hydroxyl, sulfhydryl or carboxylic acid groups on the tissue. The scaffold may be reacted with a crosslinker which reacts with amine, hydroxyl, sulfhydryl, or carboxylic acid groups on the tissue. The molecular weight of the crosslinker may be between 1 dalton and 100 megadalton. The scaffold may be reacted with amine, hydroxyl, sulfhydryl, or carboxylic acid groups on the tissue through a polymerization reaction creating polymer crosslinkers. The scaffold may comprise woven, knitted or non-woven fibers having a length between 100nm - 1m. The density of the fibrous material in the scaffold may be between 10 and 10,000 mg/cc. The porosity of the fibrous scaffold may be between 10 and 99%.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/-10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

### NUMBERED EMBODIMENTS

1. A method of making a reinforced engineered hide, the method comprising:
   placing a first plurality of collagen-releasing cells on a first surface of a mesh, the mesh having a first surface and a second surface;
   culturing the first plurality of collagen-releasing cells to form a first layer of collagen covering the first surface;
   flipping the mesh over and placing a second plurality of collagen-releasing cells onto the second surface of the mesh; and
   culturing the second plurality of collagen-releasing cells to form a second layer of collagen covering the second surface, wherein the mesh is embedded between the first and second layers of collagen and the first and second layers of collagen are connected to each other through the mesh.
2. The method of embodiment 1, wherein placing the first plurality of collagen-releasing cells comprises placing the first plurality of collagen-releasing cells on the first surface of the mesh wherein the mesh is releasably held in a frame.
3. The method of embodiment 1, further comprising flipping the mesh over so that the second surface is facing up and placing at least one first additional plurality of collagen-releasing cells atop the second surface and culturing the at least one first additional plurality of collagen-releasing cells to form at least one first additional layer of collagen covering the second surface.
4. The method of embodiment 1, further comprising flipping the mesh over so that the first surface is facing up and placing at least one second additional plurality of collagen-releasing cells atop the first surface and culturing the at least one second additional plurality of collagen-releasing cells to form at least one second additional layer of collagen over the second surface.
5. The method of embodiment 1, further comprising sequentially flipping the mesh over and placing at least one additional plurality of collagen-releasing cells atop the first surface or second surface and culturing the at least one second additional plurality of collagen-releasing cells to form at least one additional layer of collagen over the first or second surface.
6. The method of embodiment 1, further comprising removing the mesh from a frame.
7. The method of embodiment 1, further comprising tanning the mesh embedded between the first and second layers of collagen.
8. The method of embodiment 1, further comprising tanning the mesh embedded between the first and second layers of collagen with mesh removably held by a frame, wherein the mesh was secured to the frame during the step of placing the first plurality of collagen-releasing cells on a first surface of the mesh.
9. The method of embodiment 1, wherein placing the first plurality of collagen-releasing cells on the first surface comprises placing a sheet of collagen and a plurality of collagen-releasing cells on the first surface.
10. The method of embodiment 1, further comprising placing the mesh onto a preliminary layer of collagen-releasing cells in a culture dish so that the second surface of the mesh rests over the preliminary layer prior to placing the first plurality of collagen-releasing cells on the first surface of the mesh.
11. The method of embodiment 1, further comprising placing a plug against the second surface of the mesh before placing the first plurality of collagen-releasing cells on the first surface of the mesh.
12. A method of making a reinforced engineered hide, the method comprising:
   placing a first plurality of collagen-releasing cells on a first surface of a mesh, the mesh having a first surface and a second surface;
   culturing the first plurality of collagen-releasing cells to form a first layer of collagen covering the entire first surface;
   flipping the mesh over and placing a second plurality of collagen-releasing cells onto the second surface of the mesh;
   culturing the second plurality of collagen-releasing cells to form a second layer of collagen covering the entire second surface, wherein the mesh is embedded between the first and second layers of collagen and the first and second layers of collagen are connected to each other through the mesh; and
   sequentially forming additional layers of collagen over the first, second or first and second surfaces of the mesh to form a reinforced engineered hide having the mesh sandwiched between a plurality of layers of collagen-dense regions.
13. The method of embodiment 12, wherein sequentially forming additional layers of collagen comprises forming a reinforced engineered hide having the striated pattern including a plurality of layers of collagen-dense regions and at least one mesh layer.
14. A method of making a reinforced engineered hide, the method comprising:
   placing a first plurality of collagen-releasing cells on a first surface of a mesh, the mesh having a first surface and a second surface, wherein the mesh is releasably held in a frame;
   culturing the first plurality of collagen-releasing cells to form a first layer of collagen covering the first surface;
   flipping the frame and the mesh over and placing a second plurality of collagen-releasing cells onto the second surface of the mesh;
   culturing the second plurality of collagen-releasing cells to form a second layer of collagen covering the second surface so that the mesh is embedded between the first and second layers of collagen and the first and second layers of collagen are connected to each other through the mesh;
   placing at least one first additional plurality of collagen-releasing cells atop the second surface and culturing the at least one first additional plurality of collagen-releasing cells to form at least one first additional layer of collagen covering the second surface;
   flipping the frame and mesh over and placing at least one second additional plurality of collagen-releasing cells atop the first surface and culturing the at least one second additional plurality of collagen-releasing cells to form at least one second additional layer of collagen over the second surface; and
   removing the mesh from the frame.
15. A reinforced engineered hide, the hide comprising:
   a body extending in a plane and having a volume normal to the plane, the volume comprising a plurality of layers of collagen-dense regions extending in the plane within the volume to form a stratified pattern of collagen dense-regions; and
   a reinforcing mesh within the planar body and extending in the plane of the body, wherein a first layer of the plurality of layers of collagen-dense regions is adjacent to the mesh on a first side and follows a contour of the first side of the mesh and a second layer of the plurality of layers of collagen-dense regions is adjacent to the mesh on a second side and follows a contour of the second side of the mesh.
16. The reinforced engineered hide of embodiment 15, wherein the hide is tanned.
17. The reinforced engineered hide of embodiment 15, wherein the hide is substantially free of non-differentiated keratinocytes or epithelial cells.
18. The reinforced engineered hide of embodiment 15, wherein the thickness of each layer of collagen-dense region is between about 50 µm to about 200 µm.
19. The reinforced engineered hide of embodiment 15, wherein the thickness of each layer of collagen-dense region is between about 50 µm to about 150 µm.
20. The reinforced engineered hide of embodiment 15, wherein the mesh comprises a pore size of between about 2 µm to 5 mm.
21. The reinforced engineered hide of embodiment 15, further comprising one or more colorants or pigments.
22. The reinforced engineered hide of embodiment 15, wherein the mesh comprises a flexible net-like structure having a pore size of less than 5 mm.
23. The reinforced engineered hide of embodiment 15, wherein the mesh comprises a synthetic polymeric material.
24. A method of forming a fiber-reinforced biological tissue composite, the method comprising:
   culturing cells on a fibrous scaffold until fibers of the fibrous scaffold are surrounded by tissue comprising extracellular matrix, forming an intermediate tissue construct; and
   tanning the intermediate tissue construct to cross-link the fibers of the fibrous scaffold to the tissue.
25. The method of embodiment 24, wherein culturing comprises culturing cells on a fibrous silk scaffold.
26. The method of embodiment 24, wherein culturing comprises culturing cells on a fibrous protein scaffold.
27. The method of embodiment 26, wherein the fibrous protein scaffold comprises one or more of: a fibrous wool scaffold, a fibrous silk scaffold or a fibrous collagen scaffold.
28. The method of embodiment 24, wherein culturing comprises culturing cells on a protein fiber scaffold comprising amine, carboxylic acid and sulfhydryl groups.
29. The method of embodiment 24, wherein culturing comprises culturing cells on a fibrous cellulosic scaffold.
30. The method of embodiment 29, wherein the fibrous cellulosic scaffold comprises one or more of a fibrous flax scaffold, a fibrous pineapple scaffold, or a fibrous cotton scaffold.
31. The method of embodiment 24, wherein culturing comprises culturing cells on a synthetic fiber scaffold with a cross linkable surface chemistry.
32. The method of embodiment 31, wherein the fibrous synthetic scaffold comprises one or more of a carbon fiber scaffold, a Kevlar scaffold or a fiberglass scaffold.
33. The method of embodiment 24, wherein culturing comprises culturing the cells on the fibrous scaffold until the fibers are surrounded by collagen.
34. The method of embodiment 24, wherein culturing comprises culturing cells on the fibrous scaffold comprising fibers having a length of between about 100 nm and 1 m.
35. The method of embodiment 24, wherein culturing comprises culturing cells on the fibrous scaffold having a porosity of between 10 and 99%.
36. The method of embodiment 24, wherein culturing comprises culturing mammalian cells on the fibrous scaffold.
37. The method of embodiment 24, wherein tanning comprises crosslinking amine, hydroxyl, sulfhydryl, tyrosyl, and carboxylic acid groups on the extracellular matrix proteins in the tissue to the scaffold.
38. The method of embodiment 24, wherein tanning comprises crosslinking the fibers of the fibrous scaffold to the tissue through covalent bonds.
39. The method of embodiment 24, wherein tanning comprises crosslinking the fibers of the fibrous scaffold to the tissue through ionic, hydrophobic and van der Waals interactions.
40. A fiber-reinforced biological tissue composite material, the material comprising:
   a fibrous scaffold comprising a plurality of fibers, wherein the fibers are surrounded by extracellular matrix, wherein the extracellular matrix and plurality of fibers are cross-linked to each other.
41. The material of embodiment 40, wherein the scaffold is a naturally occurring protein fiber containing amine hydroxyl, sulfhydryl, tyrosyl, and carboxylic acid groups.
42. The material of embodiment 40, wherein the scaffold is a naturally occurring cellulosic fiber containing amine, hydroxyl and carboxylic acid groups.
43. The material of embodiment 40, wherein the scaffold is a synthetic fiber containing surface chemistries to crosslink to the ECM
44. The material of embodiment 40, wherein the fibers are chemically modified to enhance tissue crosslinking to scaffold.
45. The material of embodiment 40, wherein the scaffold is chemically modified to contain tissue crosslinking groups including one or more of: amines, carboxylic acids, sulfates, aldehydes, hydrazides, sulfhydryls, diazirines, aryl-azides, acrylates and epoxides.
46. The material of embodiment 45, wherein the tissue crosslinking groups are protected during tissue growth and activated for crosslinking when tissue growth is complete.
47. The material of embodiment 45 wherein the tissue crosslinking groups are pendant to the scaffold with a spacer between 1 dalton and 100 megadaltons.
48. The material of embodiment 40, wherein the scaffold is crosslinked to the tissue through non-covalent interactions including ionic, hydrophobic and van der Waals interactions.
49. The material of embodiment 40, wherein the scaffold is crosslinked to the tissue through covalent bonds.
50. The material of embodiment 40, wherein the scaffold is directly reacted with amine, hydroxyl, sulfhydryl or carboxylic acid groups on the tissue.
51. The material of embodiment 40, wherein the scaffold is reacted with a crosslinker which reacts with amine, hydroxyl, sulfhydryl, or carboxylic acid groups on the tissue.
52. The material of embodiment 51, wherein a molecular weight of the crosslinker is between 1 dalton and 100 megadalton.
53. The material of embodiment 40, wherein the scaffold is reacted with amine, hydroxyl, sulfhydryl, or carboxylic acid groups on the tissue through a polymerization reaction creating polymer crosslinkers.
54. The material of embodiment 40, wherein the scaffold comprises woven, knitted or non-woven fibers having a length between 100nm - 1m.
55. The material of embodiment 40, wherein a density of fibrous material in the scaffold is between 10 and 10,000 mg/cc.
56. The material of embodiment 40, wherein a porosity of the fibrous scaffold is between 10 and 99%

## Claims

1. A fiber-reinforced biological tissue composite material, the material comprising:
a fibrous scaffold comprising a plurality of fibers,
wherein the fibers are surrounded by extracellular matrix, and
wherein the extracellular matrix and plurality of fibers are crosslinked to each other.

2. The fiber-reinforced biological tissue composite material of claim 1, wherein the fibers are surrounded by extracellular matrix released from cells cultured on the scaffold.

3. The fiber-reinforced biological tissue composite material of claim 1 or 2, wherein the extracellular matrix defines a plurality of layers of extracellular matrix disposed over the scaffold, preferably wherein the plurality of layers is derived from cells sequentially cultured on the scaffold.

4. The fiber-reinforced biological tissue composite material of claim 3, wherein the plurality of layers comprise strata of dense regions alternating with less-dense regions;

5. The fiber-reinforced biological tissue composite material of claim 1, wherein the composite material is substantially free of non-differentiated keratinocytes or epithelial cells.

6. The fiber-reinforced biological tissue composite material of claim 1, wherein each of the fibers of the plurality of fibers has a length in a range of from 100 nm to 1 m, preferably from 100 nm to 1 mm.

7. The fiber-reinforced biological tissue composite material of claim 1, wherein the fibers of the plurality of fibers comprises at least one of woven fibers, knitted fibers, or non-woven fibers.

8. The fiber-reinforced biological tissue composite material of claim 1, wherein the fibrous scaffold has a porosity in a range of from 10% to 99%; or wherein the fibrous scaffold has a density within a range of 10 mg/cc to 10,000 mg/cc.

9. The fiber-reinforced biological tissue composite material of claim 1, wherein the fibrous scaffold has a thickness in a range of from 10 µm to 5 mm.

10. The fiber-reinforced biological tissue composite material of claim 1, wherein the fibrous scaffold comprises (i) a fibrous synthetic scaffold, preferably a material selected from the group consisting of carbon fiber, Kevlar and fiberglass; (ii) a fibrous cellulosic scaffold, preferably a material selected from the group consisting of fibrous flax, fibrous pineapple and fibrous cotton; or (iii) a fibrous protein scaffold, preferably selected from the group consisting of fibrous wool, fibrous silk, and fibrous collagen.

11. The fiber-reinforced biological tissue composite material of claim 1, wherein the fibrous scaffold comprises a naturally occurring protein fiber, the naturally occurring protein fiber comprising at least one of an amine hydroxyl group, a sulfhydryl group, a tyrosyl group, or a carboxylic acid group.

12. The fiber-reinforced biological tissue composite material of claim 1, wherein the fibrous scaffold is a synthetic fiber scaffold having a surface chemistry configured to enable cross-linking between the fibers within the plurality of fibers and the extracellular matrix.

13. The fiber-reinforced biological tissue composite material of claim 1, wherein the fibers of the plurality of fibers are chemically modified to enhance tissue crosslinking to the fibrous scaffold.

14. The fiber-reinforced biological tissue composite material of claim 1, wherein the fibrous scaffold is chemically modified to contain a tissue crosslinking group selected from the group consisting of amines, carboxylic acids, sulfates, aldehydes, hydrazides, sulfhydryls, diazirines, aryl-azides, acrylates, and epoxides.

15. The fiber-reinforced biological tissue composite material of claim 1, wherein the extracellular matrix and plurality of fibers are crosslinked to each other through covalent bonds or through non-covalent interactions, wherein the non-covalent interactions are selected from ionic interactions, hydrophobic interactions, and van der Waals interactions.
